# EUROPEAN PATENT APPLICATION

(11) **EP 0 806 480 A2**
(43) Date of publication of application: **12.11.1997**
(21) Application number: 97107329.1
(22) Date of filing: 02.05.1997
(51) Int. Cl.: C12N 15/52, C12N 15/76, C12N 1/21, C12N 9/00, C07D 493/04

(54) **Frenolicin gene cluster**

(30) Priority: 07.05.1996 US 16753; 04.04.1997 US
(71) Applicant: F. HOFFMANN-LA ROCHE AG, 4070 Basel (CH)
(72) Inventor: Reeves, Christopher Desmond, Woodinville, WA 98072 (US); Soliday, Charles Leslie, Duvall, WA 98019 (US)
(74) Representative: Braun, Axel

(57) **Abstract**

The present invention provides DNA sequences comprising DNA sequences encoding enzymes which catalyze the biosynthesis of frenolicins in prokaryotic cells, vectors comprising such DNA sequences, proteins encoded by such DNA sequences, host cells transformed by such DNA sequences and process using such transformed cells for the production of frenolicins, especially frenolicin B.

SEQUENCE LISTING

(1) GENERAL INFORMATION:

(i) APPLICANT:
(A) NAME: F.HOFFMANN-LA ROCHE AG
(B) STREET: Grenzacherstrasse 124
(C) CITY: Basle
(D) STATE: BS
(E) COUNTRY: Switzerland
(F) POSTAL CODE (ZIP): CH-4002
(G) TELEPHONE: 061 - 688 25 05
(H) TELEFAX: 061 - 688 13 95
(I) TELEX: 962292/965542 hlr ch

(ii) TITLE OF INVENTION: Frenolicin Gene Cluster

(iii) NUMBER OF SEQUENCES: 22

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(2) INFORMATION FOR SEQ ID NO:1:

(i) SEQUENCE CHARACTERISTICS:
(A) LENGTH: 770 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:1:

Met Ala Gly Ser Gly Tyr Ala Tyr Tyr Gln His Leu Ser Gly Asn Ile
1               5                   10                  15

Asp Lys Ile Asp Val Gly Asp Ala Gly Asn Lys Asp Ala Ala Pro Asp
            20                  25                  30

Gly Pro Ile Asn Ile Leu Ile Ile Gly Thr Asp Lys Arg Thr Gly Lys
        35                  40                  45

Gly Asn Glu Gly Cys Gly Gly Lys Asp Ser Pro Gly His Ala Asp Thr
    50                  55                  60

Asn Ile Leu Leu Arg Val Ser Ala Asp Arg Thr Asn Thr Thr Gly Leu
65                  70                  75                  80

Ser Ile Pro Arg Asp Leu Ile Thr Asn Ile Pro Asp Cys Leu Thr Thr
                85                  90                  95

Gln Asp Asp Gly Ser Lys Lys Thr Ile Pro Gly Thr Gln Asn Val Arg
            100                 105                 110

Phe Asn Thr Ser Leu Gly Gln Glu Gly Arg Asp Pro Gly Cys Thr Met
        115                 120                 125

Arg Thr Val Thr Glu Leu Thr Gly Leu Lys Val Asp His Phe Met Met
    130                 135                 140

Ala Asp Phe Asn Ala Val Lys Asn Leu Thr Thr Ala Val Asn Gly Val
145                 150                 155                 160

Glu Val Cys Val Ala Lys Asp Val Asp Asp Pro Asp Ser His Leu Lys
                165                 170                 175

Leu Ser Ala Gly Thr His Lys Val Gln Gly Glu Gln Ala Leu Ala Phe
            180                 185                 190

Val Arg Thr Arg His Ser Phe Gly Asn Gln Gly Asp Leu Asp Arg Ile
        195                 200                 205

Lys Val Gln Gln Gln Phe Leu Gly Ser Leu Ala Arg Gln Leu Lys Ser
    210                 215                 220

Glu Asp Thr Leu Thr Ser Pro Lys Lys Leu Tyr Lys Val Ala Glu Ala
225                 230                 235                 240

Ala Thr Asp Ala Leu Thr Val Asp Ser Gly Ile Gly Ser Ile Thr Lys
                245                 250                 255

Leu Met Ser Leu Ala Lys Glu Leu Gln His Ile Asn Pro Lys Asn Ile
            260                 265                 270

Thr Phe Val Thr Leu Pro Val Val Asp Asn Pro Ala Glu Lys Val Lys
        275                 280                 285

Ala Thr Val Val Leu Asn Glu Thr Asp Ala Asp Pro Gln Gln Ser Ala
    290                 295                 300

Leu Gly Gln Ser Leu Asp Val Gly Arg Gln Leu Val Asp Ser Leu Thr
305                 310                 315                 320

Asp Gln Asp Pro Arg Asp Gly Lys Thr Val Pro Trp Leu Ala Thr Arg
                325                 330                 335

Trp Lys Ala Asp Pro Glu Ala Thr Arg Phe Thr Phe Thr Leu Arg Ala
            340                 345                 350

Gly Ala Thr Phe Ser Asp Gly Thr Pro Val Asp Ala Arg Ala Val Lys
        355                 360                 365

Ala Asn Phe Asp Ala Val His Glu Leu Gly Ala Ala Ala Ser Arg Gly
    370                 375                 380

Ala Val Tyr Leu Asp Gly Tyr Arg Glu Thr Arg Val Ser Gly Ala Arg
385                 390                 395                 400

Thr Leu Thr Val Val Phe Asp Lys Pro Asn Ala Gln Phe Leu Arg Gly
                405                 410                 415

Thr Ser Thr Val Ser Leu Gly Leu Leu Ser Pro Gly Ser Leu Arg Arg
            420                 425                 430

Thr Pro Gln Glu Arg Cys Thr Gly Arg Leu Val Gly Ser Gly Pro Phe
        435                 440                 445

Val Leu Asp Arg Tyr Arg Pro Asn Thr Ser Val Thr Leu Asp Arg Arg
    450                 455                 460

Lys Gly Tyr Ser Trp Gly Ser Arg Leu Trp Gln Arg Glu Gly Gly Ala
465                 470                 475                 480

Tyr Leu Glu Gly Val Glu Tyr Arg Ile Val Pro Glu Asn Thr Thr Arg
                485                 490                 495

Ser Gly Ala Leu Ser Ala Gly Gln Leu Asp Val Ala Thr Ala Leu Ala
            500                 505                 510

Pro Gln Asp Arg Glu Arg Phe Ser Ala Pro Gly Trp Ser Leu Leu Thr
        515                 520                 525

Arg Thr Ala Pro Gly Val Asp Leu Ser Leu Tyr Val Asn Ala Arg Arg
    530                 535                 540

Thr Ala Leu Arg Glu Ala Ala Val Arg Gln Ala Leu Gln Lys Gly Ile
545                 550                 555                 560

Asp Arg Glu Ala Val Ala Thr Thr Phe Leu Ser Ser Arg Lys Leu Ala
                565                 570                 575

Ala Thr Ser Val Leu Ser Ser Thr Thr Pro Gly Tyr Thr Asp Leu Gly
            580                 585                 590

Asp Arg Leu Ala His Asp Pro Ala Gly Ala Arg Arg Leu Leu Asp Lys
        595                 600                 605

Ala Gly Trp Arg Pro Gly Ala Asp Gly Ile Arg Val Lys Asn Gly Val
    610                 615                 620

Arg Leu Arg Leu Asp Ala Val Phe Val Arg Gln Gln Ser Leu Glu Leu
625                 630                 635                 640

Val Gln Gln Gln Leu Lys Asp Ile Gly Val Glu Leu Arg Leu Arg Gln
                645                 650                 655

Leu Thr Val Ser Arg Phe Pro Glu Val Leu Ala Ala Gly Ser Tyr Asp
            660                 665                 670

Leu Ser Leu Gln Ser Ala Asn Arg Ala Asp Pro Asp Val Leu Thr Thr
        675                 680                 685

Ala Phe Ala Gly Gly Thr Pro Val Ala Asp Ala Arg Leu Arg Ser Glu
    690                 695                 700

Leu Arg Arg Ala Thr Ser Ser Thr Asp Glu Ala Thr Arg Ser Ser Leu
705                 710                 715                 720

Phe Ala Ala Ala Gln Arg Arg Leu Val Asp Glu Gly His Val Leu Pro
                725                 730                 735

Leu Asn Glu Thr Glu Glu Thr Ala Ala Leu Ser Thr Arg Val His Gly
            740                 745                 750

Leu Thr Arg Asp Ala Ser Asn Arg Leu Val Leu His Asp Thr Trp
        755                 760                 765

Thr Thr Gly
        770

(2) INFORMATION FOR SEQ ID NO:2:

      (i) SEQUENCE CHARACTERISTICS:
           (A) LENGTH: 323 amino acids
           (B) TYPE: amino acid
           (C) STRANDEDNESS: single
           (D) TOPOLOGY: linear

     (ii) MOLECULE TYPE: protein

    (iii) HYPOTHETICAL: NO

     (iv) ANTI-SENSE: NO

     (xi) SEQUENCE DESCRIPTION: SEQ ID NO:2:

Met Thr Ala Arg Tyr Leu Ala Arg Arg Leu Gly Arg Val Val Leu Val
1               5                   10                  15

Val Trp Ala Ala Tyr Thr Leu Ser Phe Ala Val Leu Tyr Leu Leu Pro
            20                  25                  30

Gly Asp Pro Val Gln Thr Met Leu Ser Gly Ala Ala Gly Gly Asp Gly
        35                  40                  45

Ala Ala Val Asp Pro His Glu Ala Gln Arg Leu Arg His Thr Leu Gly
    50                  55                  60

Leu Asp Arg Pro Leu Ala Val Gln Tyr Thr Ser Met Leu Gly His Ala
65                  70                  75                  80

Leu Arg Gly Asp Leu Gly Thr Ser Ile Arg Ser Gly Ala Pro Val Arg
                85                  90                  95

Gly Gln Leu Ala Gln Ala Leu Pro Asp Thr Leu Ser Val Ala Leu Pro
            100                 105                 110

Ala Leu Val Leu Ser Val Leu Val Ala Leu Cys Leu Ala Leu Leu Gly
        115                 120                 125

Ala Trp Pro Arg Arg Arg Ala Leu Arg Arg Ala Ala Thr Ala Leu Pro
    130                 135                 140

Ser Leu Gly Thr Ala Met Pro Ser Phe Trp Leu Gly Leu Leu Leu Ala
145                 150                 155                 160

Gln Trp Val Ser Phe Arg Trp Gly Leu Leu Pro Ala Thr Gly Gly Gly
                165                 170                 175

Arg Ser Pro Arg Ala Thr Leu Leu Ala Ala Leu Thr Leu Ala Leu Pro
            180                 185                 190

Ile Gly Cys Val Leu Ala Gln Val Leu Gly Arg Gly Leu Arg Ala Ala
        195                 200                 205

Leu Ala Glu Pro Tyr Ala Asp Val Ala Arg Ser Arg Gly Ala Gly Arg
    210                 215                 220

Ala Arg Leu Leu Leu Ala Arg Ala Leu Arg Asn Ala Ser Val Ala Ala
225                 230                 235                 240

Leu Ala Leu Leu Gly Val Val Cys Gly Gln Leu Leu Ala Gly Ala Val
                245                 250                 255

Leu Val Glu Thr Val Phe Ala Arg Gly Gly Ile Gly Arg Leu Ala Met
            260                 265                 270

Asp Ala Val Thr Tyr Gln Asp Leu Pro Val Val Gln Gly Val Val Val
        275                 280                 285

Leu Ala Ala Leu Val Ala Ala Leu Val Asn Leu Val Val Asp Leu Leu
    290                 295                 300

Leu Pro Leu Leu Glu Pro Arg Thr Ala Ser Glu Ala Ala Asp Ala Val
305                 310                 315                 320

Pro Ala His

(2) INFORMATION FOR SEQ ID NO:3:

     (i) SEQUENCE CHARACTERISTICS:
          (A) LENGTH: 274 amino acids
          (B) TYPE: amino acid
          (C) STRANDEDNESS: single
          (D) TOPOLOGY: linear

    (ii) MOLECULE TYPE: protein

   (iii) HYPOTHETICAL: NO

    (iv) ANTI-SENSE: NO

    (xi) SEQUENCE DESCRIPTION: SEQ ID NO:3:

Met Ala Leu Arg Arg Val Ala Ala Leu Trp Arg Ala Pro Gly Leu Ala
1               5                   10                  15

Leu Ser Leu Leu Val Leu Leu Leu Val Leu Gly Trp Ala Leu Leu Pro
            20                  25                  30

Gly Leu Phe Thr Ala Ala Asp Pro Leu Arg Ala Asp Ala Ala His Arg
        35                  40                  45

Leu Leu Ala Pro Gly Ala Gly His Pro Leu Gly Ala Asp His Val Gly
    50                  55                  60

Arg Asp Leu Tyr Ala Arg Val Val His Gly Thr Ala Arg Ser Leu Gly
65                  70                  75                  80

Thr Ala Phe Ala Ala Val Ala Leu Gly Val Leu Ala Gly Gly Ala Leu
                85                  90                  95

Gly Ala Val Ala Gly Val Ala Gly Arg Ala Val Asp Ala Val Val Met

            100                 105                 110

Arg Val Val Asp Val Leu Leu Ala Val Pro Gly Leu Leu Leu Ser Leu
        115                 120                 125

Ala Val Val Ser Ala Leu Gly Phe Gly Thr Ala Gln Val Ala Cys Ala
    130                 135                 140

Val Gly Val Gly Thr Val Gly Gly Ile Ala Arg Val Ser Arg Ala Gln
145                 150                 155                 160

Val Arg Arg Val Arg Gly Gly Glu Tyr Val Glu Ala Ala Arg Leu Ala
                165                 170                 175

Gly Val Ala Gly Pro Leu Ile Leu Leu Arg His Ile Val Pro Asn Ala
            180                 185                 190

Ala Pro Pro Val Leu Ala Leu Ala Val Thr Glu Cys Gly Thr Ala Val
        195                 200                 205

Leu Gly Val Ala Ser Leu Gly Phe Leu Gly Phe Gly Ala Pro Pro Pro
    210                 215                 220

Ala Pro Glu Trp Gly Ala Leu Ile Ser Thr Gly Arg Asp Tyr Leu Val
225                 230                 235                 240

Ser Ala Trp Trp Leu Thr Thr Leu Pro Gly Leu Val Leu Val Ala Leu
                245                 250                 255

Val Val Ala Leu His Arg Val Gly Arg Ala Leu Glu Arg Glu Glu Arg Thr
Gly
260 265 270

(2) INFORMATION FOR SEQ ID NO:4:

(i) SEQUENCE CHARACTERISTICS:
(A) LENGTH: 524 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:4:

Met Thr Pro Ala Asp Lys Pro Thr Asp Glu Arg Ser Pro Val Leu Asp
1               5                   10                  15

Leu Ser Gly Val Ser Val Ala Tyr Gly Thr Arg Thr Val Leu His Gly
            20                  25                  30

Ile Asp Leu Arg Leu Ala Pro Gly Gln Val Leu Ala Val Leu Gly Ala
        35                  40                  45

Ser Gly Ser Gly Lys Ser Thr Leu Ala Gln Ala Ala Leu Gly Leu Leu
    50                  55                  60

Pro Pro Gly Gly Arg Val Thr Ala Gly Arg Val Thr Val Ala Gly His
65                  70                  75                  80

Asp Ile Thr Ala Leu Ala Pro His Arg Leu Arg Ala Leu Arg Gly Thr
                85                  90                  95

Val Thr Gly Leu Val Pro Gln Asp Gln Ala Val Ser Leu Asp Pro Leu
            100                 105                 110

Val Arg Val Gly Ala Gln Val Thr Glu Thr Leu Arg Ala His Arg Leu
        115                 120                 125

Glu Asp Arg Arg Glu Ala Ala Arg Arg Ala Val Pro Leu Leu Gly Glu
    130                 135                 140

Ala Gly Ile Glu Ala Pro Gly Pro Leu Ala Arg Ala Tyr Pro His Ala
145                 150                 155                 160

Leu Ser Gly Gly Gln Arg Gln Arg Val Leu Val Ala Gly Ala Phe Ala
                165                 170                 175

Ala Arg Pro Pro Leu Val Val Ala Asp Glu Pro Thr Ser Ala Leu Asp
            180                 185                 190

Ala Thr Val Arg Arg Arg Val Met Asp Arg Phe Ala Ala Leu Val Ala
        195                 200                 205

Ala His Gly Thr Ala Val Leu Leu Val Thr His Asp Phe Arg Leu Ala
    210                 215                 220

Arg Glu Arg Ala Asp Gln Val Ala Val Leu Ala Asp Gly Arg Leu Val
225                 230                 235                 240

Glu Ser Gly Pro Ala Ala Arg Val Leu Asp Arg Pro Ala His Pro Tyr
                245                 250                 255

Thr Arg Arg Leu Thr Gly Ala Gly Arg Arg Val Ala Ala Arg Gly Thr
            260                 265                 270

Ala Pro Arg Ala Ser Gly Thr Pro Val Val Arg Ala Arg Asp Leu Val
        275                 280                 285

Lys Glu Tyr Arg Arg Asp Gly Arg Arg Val Arg Ala Val Asp Gly Val
    290                 295                 300

Gly Phe Thr Val Arg Glu Gly Glu Phe Phe Ala Leu Val Gly Glu Ser
305                 310                 315                 320

Gly Ser Gly Lys Ser Thr Thr Ala Arg Leu Val Thr Gly Leu Thr Ala
                325                 330                 335

Pro Thr Ser Gly Ala Val Glu His Ala Pro Ala Pro Val Arg Pro Gln
            340                 345                 350

Leu Val Gln Gln Ser Pro Tyr Ala Ala Phe Asp Pro Arg Trp Thr Val
        355                 360                 365

Arg Arg Ile Val Glu Glu Pro Leu Arg Ala Arg His Val Pro Gly Ala
    370                 375                 380

Arg Arg Arg Ala Arg Leu Arg Glu Leu Leu Ala Leu Val Gly Leu Asp
385                 390                 395                 400

Glu Glu Leu Leu Ala Arg Arg Pro Arg Glu Leu Ser Gly Gly Gln Arg
                405                 410                 415

Gln Arg Val Ala Leu Ala Arg Ala Leu Ala Pro Glu Pro Arg Leu Leu
            420                 425                 430

Val Cys Gly Glu Pro Val Ala Ala Leu Asp Pro Val Ala Arg Glu Arg
        435                 440                 445

Val Val His Leu Leu Glu Arg Leu Arg Ala Glu Leu Gly Leu Thr Cys
    450                 455                 460

Leu Phe Val Ser His Glu Leu Asp Val Val Arg Arg Leu Cys Gly Arg
465                 470                 475                 480

Val Ala Val Met Arg Gly Gly Arg Leu Leu Glu Ser Gly Pro Val Gly
                485                 490                 495

Glu Val Leu Ser Ala Pro Ser Gln Pro Tyr Thr Arg Ala Leu Leu Ala
            500                 505                 510

Ala Glu Ala Gly Pro Ser Asp Thr Pro Gly Ala Gly
        515                 520

(2) INFORMATION FOR SEQ ID NO:5:

(i) SEQUENCE CHARACTERISTICS:
(A) LENGTH: 216 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:5:

Met Asn Glu Ile Thr Val Glu Ile Trp Thr Asp Val Val Cys Pro Trp
1               5                   10                  15

Cys Tyr Ile Gly Lys Arg Arg Phe Glu Arg Ala Leu Ala Ala Phe Asp
            20                  25                  30

Ala Lys Glu Asp Val Arg Val His Trp Arg Ser Phe Glu Leu Asp Pro
        35                  40                  45

Ala Ala Leu Arg Val Thr Asp Glu Thr Ile Pro Glu Arg Met Leu Arg
    50                  55                  60

Arg Gln Gly Ile Pro Pro Glu Gln Ala Ala Glu Leu Leu Ala Gly Val
65                  70                  75                  80

Ser Ala Gln Ala Glu Ala Glu Gly Leu Glu Tyr His Leu Asp Arg Ala
                85                  90                  95

Arg Pro Cys Asn Thr Phe Asp Ala His Arg Leu Ala His His Ala Gly
            100                 105                 110

Thr Arg Gly Leu Ala Glu Thr Phe Gln Glu Arg Leu Met Cys Ala Tyr
        115                 120                 125

Thr Ala Glu Gly Val Ser Val Gly Asp His Pro Thr Leu Leu Ala Leu
    130                 135                 140

Ala Glu Glu Ala Gly Leu Asp Ala Ala Ala Ala Ala Glu Val Leu Ala
145                 150                 155                 160

Gly Asp Ala His Ala Glu Asp Val Arg Ala Asp Glu Asp Arg Ala Ala
                165                 170                 175

Arg Leu Gly Val Gly Gly Val Pro Ala Phe Val Ile Gly Gly Arg Trp
            180                 185                 190

Ser Val Ser Gly Ala Gln Pro Ala Glu Leu Leu Thr Gly Leu Leu Glu
        195                 200                 205

Arg Ala Arg Thr Ala Ala Ala Ala
    210                 215

(2) INFORMATION FOR SEQ ID NO:6:

     (i) SEQUENCE CHARACTERISTICS:
          (A) LENGTH: 517 amino acids
          (B) TYPE: amino acid
          (C) STRANDEDNESS: single
          (D) TOPOLOGY: linear

    (ii) MOLECULE TYPE: protein

   (iii) HYPOTHETICAL: NO

    (iv) ANTI-SENSE: NO

    (xi) SEQUENCE DESCRIPTION: SEQ ID NO:6:

Met Ser Ser Ser Pro Pro Ala Pro Ala Thr Pro Gly Val Ala Pro His
1               5                   10                  15

Ser Pro Pro Ala Pro Arg Leu Gly Leu Val Leu Leu Val Cys Cys Leu
            20                  25                  30

Ala Gln Phe Leu Val Thr Leu Ser Val Ala Ile Val Asn Val Ala Leu
        35                  40                  45

Pro Asp Ile Gln Arg Gly Leu Gly Phe Ser Ala Glu Ser Leu Gln Trp
    50                  55                  60

Val Val Asn Ala Tyr Thr Val Thr Phe Ala Gly Phe Leu Leu Leu Gly
65                  70                  75                  80

Gly Arg Ile Ala Asp Leu Phe Gly Arg Arg Arg Ile Phe Leu Ala Gly
                85                  90                  95

Val Ala Leu Phe Ala Leu Ala Ser Leu Ala Gly Gly Leu Ser Gln Asn
            100                 105                 110

Ala Gly Thr Leu Val Ala Ala Arg Ala Val Gln Gly Leu Ala Ala Ala
        115                 120                 125

Val Ile Ala Pro Thr Thr Leu Ala Val Leu Gly Thr Ser Phe Lys Asp
    130                 135                 140

Pro His Gln Arg His Arg Ala Phe Gly Ala Trp Gly Ala Val Ser Gly
145                 150                 155                 160

Ala Gly Gly Ala Phe Gly Ala Leu Ala Gly Gly Ala Leu Thr Asp Ala
                165                 170                 175

Phe Ser Trp Arg Trp Val Leu Phe Val Asn Leu Pro Ile Gly Val Leu
            180                 185                 190

Leu Leu Ala Gly Ile Ala Trp Gly Ile Ser Glu Leu Arg His Ala Gly
        195                 200                 205

Glu Asp Arg Arg Ile Asp Val Ala Gly Ala Leu Thr Val Thr Leu Gly
    210                 215                 220

Leu Leu Ala Leu Val Leu Gly Ile Val Gln Ser Gly Pro His Gly Trp
225                 230                 235                 240

Gly Ser Ala Ala Thr Leu Val Pro Leu Leu Gly Gly Leu Ala Leu Leu
                245                 250                 255

Gly Ala Phe Val Leu Val Glu Gly Arg Phe Ala Pro Gln Pro Leu Ile
            260                 265                 270

Pro Leu Gly Ile Phe Arg Ser Arg Ser Val Val Ala Ala Asn Val Val
        275                 280                 285

Ala Met Thr Ser Gly Ala Ala Leu Phe Ser Met Phe Tyr Phe Leu Thr
    290                 295                 300

Leu Phe Leu Asn Gln Val Arg Asp Tyr Ser Pro Leu Arg Thr Gly Phe
305                 310                 315                 320

Ala Tyr Leu Pro Leu Ala Leu Ala Ile Met Val Ala Ala Gln Phe Ser
                325                 330                 335

Ala Ala Leu Val Arg Val Leu Gly Pro Arg Thr Thr Leu Leu Val Ser
            340                 345                 350

Met Ala Leu Thr Ala Ala Gly Leu Leu Trp Leu Ser Arg Leu Thr Glu
        355                 360                 365

Asp Ser Gly Phe Ala Gly Gly Leu Leu Gly Pro Thr Leu Val Val Gly
    370                 375                 380

Ile Gly Gln Gly Ile Ser Met Ser Ala Ser Ala Ile Ala Gly Val Ala
385                 390                 395                 400

Gly Val Arg Pro Gln Gln Ala Gly Leu Ala Ser Gly Leu Leu Asn Ala
                405                 410                 415

Thr Arg Gln Leu Gly Gly Ala Leu Gly Leu Ala Val Val Ala Ala Val
            420                 425                 430

Ala Thr Ser Arg Ala Asp Gly Leu Leu Asp Gly Val Ala Arg Pro Thr
        435                 440                 445

Ala Glu Leu Ala Arg His Ala Gln Ala Ser Gly His Pro Leu Ser Ile
    450                 455                 460

Ala Val Ala Ala Ala Leu Ser Ala Val Gly Leu Leu Ala Ser Leu Ala
465                 470                 475                 480

Ala Pro Gly Arg Ser Pro Ala Pro Thr Gly Thr Arg Thr Gly Gly Asp
                485                 490                 495

Ser Ala Ala Pro Ala Pro Ala Ala Ala Pro Ala Ala Thr Gly Thr Thr
            500                 505                 510

Gly Pro Gly Glu Ile
        515

(2) INFORMATION FOR SEQ ID NO:7:

(i) SEQUENCE CHARACTERISTICS:
(A) LENGTH: 282 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:7:

Met Glu Ile Lys Tyr Met Gly Gln Leu Thr Met Arg Trp Glu Gly Arg
1               5                   10                  15

Glu Lys Leu Pro Ser Ala Arg Lys Pro Arg Thr Val Leu Ala Leu Leu
            20                  25                  30

Leu Leu Asn Asp Lys Thr Pro Val Thr Thr Ser Ala Leu Ile Thr Glu
        35                  40                  45

Leu Trp Gly Glu Asn Pro Pro Arg Ser Ala Leu Thr Thr Leu Gln Thr
    50                  55                  60

Tyr Ile Leu Gln Leu Arg Lys Cys Leu Ala Ala Met Ser Gly Arg Ser
65                  70                  75                  80

Leu Ala Cys Ile Ser Glu Lys Thr Leu Val Thr Trp Pro Cys Gly Tyr
                85                  90                  95

Leu Ala Arg Leu Pro Ala Asp Ala Thr Ser Asp Val Ala Glu Phe Arg
            100                 105                 110

Arg Phe Ala Arg Glu Gly Arg Glu Ala Glu Arg Arg Gly His Leu Ala
        115                 120                 125

Glu Ala Val Arg Ser Tyr Arg Ala Ala Leu Ser Leu Ser Gln Gly Pro
    130                 135                 140

Leu Leu Ala Asp Ile Glu His Gly Pro Leu Leu Arg Ala Glu Ala Val
145                 150                 155                 160

Arg Met Glu Glu Cys Arg Leu Ser Leu Val Glu Arg Ser Ile Glu Gly
                165                 170                 175

Asp Leu Leu Leu Gly Arg His Arg Glu Val Val Ser Glu Leu Ser Ala
            180                 185                 190

Leu Val Ala Gln Tyr Pro Tyr His Glu Gln Leu Thr Gly Gln Leu Met
        195                 200                 205

Val Ala Leu Val Arg Cys Gly Arg Arg Gln Asp Ala Leu Ala Val His
    210                 215                 220

Gln Arg Leu Arg Ala Arg Met Val Glu Asp Leu Gly Leu Glu Pro Ser
225                 230                 235                 240

Ser His Leu Arg Ala Leu Gln Ser Ala Val Leu Ser Gly Glu Pro Leu
                245                 250                 255

Pro Gly Pro Pro Gly Thr Gly Gly Glu Ile Pro Thr Pro Tyr Ala Gly
            260                 265                 270

Ala Phe Ala Thr Ala Ala Arg Ser His Asp
        275                 280

(2) INFORMATION FOR SEQ ID NO:8:

(i) SEQUENCE CHARACTERISTICS:
(A) LENGTH: 171 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:8:

Met Asn Glu Thr Arg Thr Ala Ala Arg Thr Gly Gln Val Gly Pro Val
1               5                   10                  15

Asp Ala Glu Gly Phe Arg Ala Ala Met Ser Cys Phe Pro Ala Gly Val
            20                  25                  30

Val Leu Val Thr Thr Arg Glu Glu Asp Gly Thr Pro Arg Gly Phe Thr
        35                  40                  45

Ala Ser Ser Phe Cys Ser Val Ser Leu Ala Pro Pro Leu Val Ser Val
    50                  55                  60

Cys Gln Gly Thr Gly Ala Gln Ser Tyr Gly Ala Phe Gln Glu Cys Ala
65                  70                  75                  80

Arg Phe Ala Val Ser Val Leu Arg Ser Gly Gln Arg Glu Leu Ala Ser
                85                  90                  95

Arg Phe Ala Thr Arg Gly Ala Asp Lys Phe Gly Gly Gly Gly Leu Val
            100                 105                 110

Ala Leu Glu Gly Ser Gly Leu Leu Val Ala Ala Asp Ala Leu Val Thr
        115                 120                 125

Leu Glu Cys Ala Val His Ala Arg His Leu Ala Gly Asp His Val Ile
    130                 135                 140

Leu Val Gly Glu Val Arg Gly Val Gly Gln Gly Glu Gly Glu Pro Leu
145                 150                 155                 160

Val His Trp Glu Arg Gly Phe Arg Ala Leu Arg
                165                 170

(2) INFORMATION FOR SEQ ID NO:9:

(i) SEQUENCE CHARACTERISTICS:
(A) LENGTH: 336 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:9:

Met Thr Gly Glu Ala Glu Met Leu Gly Thr Arg Pro Val Val His Ser
1               5                   10                  15

Arg Leu Leu Gly Val Gly Gly Tyr Arg Pro Arg Arg Ser Val Asp Asn
            20                  25                  30

Ala Glu Leu Cys Ala Thr Val Ala Ser Thr Pro Glu Trp Ile Glu Thr
        35                  40                  45

Arg Ser Gly Ile Arg Ala Arg Gly Phe Ala Ala Pro Asp Glu Thr Leu
    50                  55                  60

Arg Phe Met Gly Arg Ala Ala Ala Glu Lys Ala Leu Ala Arg Ala Gly
65                  70                  75                  80

Val Leu Pro Asp Gly Ile Asp Leu Val Leu Val Ala Ser Met Ser Arg
                85                  90                  95

Leu Glu Gln Thr Pro Pro Leu Ala Val Leu Leu Ala Glu Asp Leu Gly
            100                 105                 110

Ala Arg Ala Ala Ala Gly Leu Asp Val Ser Gly Ala Cys Ala Gly Phe
        115                 120                 125

Cys His Ala Leu Ala Leu Ala Ser Asp Ala Val Arg Ala Gly Ser Ala
    130                 135                 140

Arg His Val Leu Val Val Gly Thr Glu Arg Met Thr Asp Leu Val Glu
145                 150                 155                 160

Arg Ala Asp Arg Thr Val Ser Val Leu Phe Ala Asp Gly Ala Gly Ala
                165                 170                 175

Ala Val Val Gly Pro Ser Ala Arg Pro Gly Ile Ser Pro Pro Ala Arg
            180                 185                 190

Gly Ala Ala Gly Arg Tyr Ala Gly Ala Leu Arg Met Asp Arg Gly Trp
        195                 200                 205

Asp Thr Phe Ala Ala Asp Pro Ser Leu Gly Arg Pro Trp Met Arg Met
    210                 215                 220

Asp Gly Arg Arg Val Phe Arg Trp Ala Met Asp Glu Val Thr Pro Arg
225                 230                 235                 240

Ala Ala Glu Leu Leu Arg Glu Ser Gly Ile Glu Pro Glu Ala Leu Asp
                245                 250                 255

Ala Phe Val Pro His Gln Ala Asn Leu Arg Met Ile Glu Leu Met Ala
            260                 265                 270

Glu Arg Leu Gly Leu Pro Glu Arg Thr Ala Val Ala Arg Asp Val Val
        275                 280                 285

Arg Ala Gly Asn Thr Ser Ala Ala Ser Val Pro Leu Ala Leu Glu Ala
    290                 295                 300

Leu Leu Asp Ser Gly Glu Val Gly Ser Gly Asp Arg Ala Leu Leu Val
305                 310                 315                 320

Gly Phe Gly Ala Gly Leu Asn Tyr Ala Ala Gln Val Val Glu Leu Pro
                325                 330                 335

(2) INFORMATION FOR SEQ ID NO:10:

     (i) SEQUENCE CHARACTERISTICS:
          (A) LENGTH: 85 amino acids
          (B) TYPE: amino acid
          (C) STRANDEDNESS: single
          (D) TOPOLOGY: linear

    (ii) MOLECULE TYPE: protein

   (iii) HYPOTHETICAL: NO

    (iv) ANTI-SENSE: NO

(xi) SEQUENCE DESCRIPTION: SEQ ID NO:10:

Met Ser Ala Phe Thr Leu Thr Glu Phe Lys Lys Leu Val Glu Gln Ser
1               5                   10                  15

Tyr Asp Ala Glu Ser Ala Glu Ala Leu His Gly Gln Ala Leu Asp Thr
            20                  25                  30

Ser Phe Thr Asp Leu Gly Tyr Asp Ser Leu Thr Val Tyr Glu Ile Val
        35                  40                  45

Thr Arg Ile Gln Asp Glu His Gly Val Thr Val Pro Asp Glu Glu Leu
    50                  55                  60

Asp Leu Leu Asp Thr Pro Arg Ala Leu Ile Ala Tyr Val Asp Ala Arg
65                  70                  75                  80

Ala Gly Ser Arg Thr
                85

(2) INFORMATION FOR SEQ ID NO:11:

(i) SEQUENCE CHARACTERISTICS:
(A) LENGTH: 351 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:11:

Met Arg Ser Glu Gly Gly Thr Glu Glu Glu Gly Ala Pro Val Val Ala
1               5                   10                  15

Leu Leu Leu Pro Gly Gln Gly Ala Gln Arg Ala Arg Met Ala Ala Gly
            20                  25                  30

Leu His Gly Val Val Pro Glu Phe Thr Thr Ala Val Glu Glu Cys Phe
        35                  40                  45

Ala Val Trp Gly Thr Trp Gly Glu Glu Leu Arg Ala Arg Trp Leu Asp
    50                  55                  60

Gly Ala Gly Gly Glu Glu Ala Leu Glu Arg Ala Ala Val Ala Gln Pro
65                  70                  75                  80

Leu Leu Phe Ala Val Gly Tyr Gly Leu Gly Arg Ala Leu Gly Ala Gly
                85                  90                  95

Ala Gln Gly Ala Pro His Leu Leu Leu Gly His Ser Val Gly Glu Leu
            100                 105                 110

Ala Ala Ala Ala Leu Ala Gly Val Cys Ala Pro Gly Ala Ala Leu Arg
        115                 120                 125

Leu Leu Ala Glu Arg Asp Ala Val Leu Arg Ala Ala Pro Ser Gly Gly
    130                 135                 140

Met Leu Ala Val Ala Ala Pro Val Asp Asp Leu Arg Pro Tyr Val Gly
145                 150                 155                 160

Ala Asp Val Val Val Gly Ala Val Asn Gly Pro Arg Gln Thr Val Leu
                165                 170                 175

Cys Gly Pro Glu Ala Pro Leu Arg Ala Val Ala Arg Arg Leu Ala Asp
            180                 185                 190

Asp Gly Leu Thr Ala Arg Arg Leu Gln Ala Asp Val Pro Phe His Ser
        195                 200                 205

Pro Ala Leu Ala Gly Ala Ala Arg Arg Leu Thr Arg Ala Ser Ala Glu
    210                 215                 220

Arg Val Ala Arg Trp Arg Pro Pro Ala Val Pro Leu Trp Ser Gly Arg
225                 230                 235                 240

Thr Gly Arg Ala Leu Thr Pro Gly Glu Ala Val Arg Ala Ala Phe Trp
                245                 250                 255

Cys Gly Gln Leu Ala Ala Pro Val Leu Tyr Trp Pro Ala Leu Gly Asn
            260                 265                 270

Leu Leu Ala Thr Ala Thr Ala Thr Ala Thr Thr Thr Gly Gly Gly Arg
        275                 280                 285

Gly Val Val Leu Leu Asp Ala Ser Pro Asp Gly Ser Leu Gly Ala Pro
    290                 295                 300

Ala Arg Arg His Pro Ala Val Arg Ser Gly Ala Ala Arg Val Val Arg
305                 310                 315                 320

Leu Leu Pro Ala Arg Pro Gly Asp Pro Ala Asp Asp Val Arg Ala Phe
                325                 330                 335

Arg Ala Ala Leu Gln Gln Ala Gly Gln Val Val Arg Asp Gly Gly
            340                 345                 350

(2) INFORMATION FOR SEQ ID NO:12:

(i) SEQUENCE CHARACTERISTICS:
(A) LENGTH: 425 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:12:

Met Asn Arg Gln Val Ala Val Thr Gly Ile Gly Val Val Ala Pro Gly
1               5                   10                  15

Gly Ile Gly Arg Lys Pro Tyr Trp Glu Gln Leu Thr Ser Gly Arg Thr
            20                  25                  30

Ala Thr Arg Ala Ile Ser Phe Phe Asp Ala Ser Pro Phe Arg Ser Arg
        35                  40                  45

Ile Ala Ala Glu Val Asp Phe Asp Pro Ala Ala Ala Gly Leu Ser Pro
    50                  55                  60

Arg Glu Val Arg Arg Met Asp Arg Ala Ala Gln Phe Ala Val Val Ser
65                  70                  75                  80

Ala Arg Glu Ser Leu Ala Asp Ser Gly Leu Asp Val Ala Asp Leu Asp
                85                  90                  95

Pro His Arg Ile Gly Val Ser Ile Gly Ser Ala Val Gly Gly Thr Thr
            100                 105                 110

Ser Leu Glu Arg Glu Tyr Leu Ala Leu Ser Asp Ser Gly Arg Gln Trp
        115                 120                 125

Glu Leu Asp Leu Ser Tyr Leu Ser Pro His Leu Tyr Asp Ala Phe Thr
    130                 135                 140

Pro Ser Ser Leu Ala Arg Glu Val Ala Gly Val Ile Gly Ala Glu Gly
145                 150                 155                 160

Pro Ala Ala Val Val Ser Thr Gly Cys Thr Ser Gly Ile Asp Ser Leu
                165                 170                 175

Gly His Ala Arg Asp Leu Ile Ala Glu Gly Ser Ala Asp Val Val Leu
            180                 185                 190

Ala Gly Gly Thr Asp Thr Pro Ile Ser Pro Ile Ala Val Ala Cys Phe
        195                 200                 205

Asp Ala Ile Lys Ala Thr Ser Pro Ser Asn Asp Asp Pro Ala His Ala
    210                 215                 220

Ser Arg Pro Phe Asp Arg Glu Arg Asn Gly Phe Val Leu Ala Glu Gly
225                 230                 235                 240

Ala Ala Val Leu Val Leu Glu Glu Leu Gly His Ala Arg Ala Arg Asp
                245                 250                 255

Ala His Val Tyr Ala Leu Val Ser Gly Tyr Ala Thr Arg Cys Asn Ala
            260                 265                 270

Tyr His Met Thr Gly Leu Thr Pro His Gly Arg Glu Met Ala Glu Ala
        275                 280                 285

Ile Arg His Ala Leu Ala Glu Ser Gly Thr Asp Pro Ala Ala Val Asp
    290                 295                 300

Tyr Val Asn Ala His Gly Ser Gly Thr Lys Gln Asn Asp Arg His Glu
305                 310                 315                 320

Thr Ala Ala Phe Lys Ala Thr Leu Gly Glu Arg Ala Arg Ser Val Pro
                325                 330                 335

Val Ser Ser Ile Lys Ser Met Ile Gly His Ser Leu Gly Ala Ile Gly
            340                 345                 350

Ser Leu Glu Ile Ala Ala Trp Ala Leu Ala Met Glu Tyr Gly Val Val
        355                 360                 365

Pro Pro Thr Ala Asn Leu Asp Thr Pro Asp Pro Glu Cys Asp Leu Asp
    370                 375                 380

Tyr Val Pro His Glu Ala Arg Glu Thr Arg Val Asp Arg Val Leu Ser
385                 390                 395                 400

Val Gly Ser Gly Phe Gly Gly Phe Gln Ser Ala Met Val Leu Thr Arg
                405                 410                 415

Asp Thr Gly Ala Arg Leu Pro Thr Ala
            420                 425

(2) INFORMATION FOR SEQ ID NO:13:

     (i) SEQUENCE CHARACTERISTICS:
          (A) LENGTH: 426 amino acids
          (B) TYPE: amino acid
          (C) STRANDEDNESS: single
          (D) TOPOLOGY: linear

    (ii) MOLECULE TYPE: protein

   (iii) HYPOTHETICAL: NO

    (iv) ANTI-SENSE: NO

    (xi) SEQUENCE DESCRIPTION: SEQ ID NO:13:

Met Thr Thr Ala Pro Ser Arg Thr Ala Gln Gly Ala Pro Pro Gly Ala
1               5                   10                  15

Ala Leu Pro Pro Val Phe Thr Gly Ile Gly Val Ala Ala Pro Asn Gly
            20                  25                  30

Leu Gly Thr Glu Glu Trp Trp Ala Ala Thr Leu Arg Gly Glu His Gly
        35                  40                  45

Leu Arg Pro Val Thr Glu Tyr Asp Ala Ser Gly His Pro Gly Gly Leu
    50                  55                  60

Val Gly Arg Val Pro Asp Phe Asp Ala Ala Arg His Leu Pro Gly Arg
65                  70                  75                  80

Leu Leu Pro Gln Thr Asp Arg Val Thr Arg Leu Ala Leu Val Ala Ala
                85                  90                  95

Asp Glu Ala Leu Lys Asp Ala Ala Val Asp Pro Ala Arg Leu Pro Glu
            100                 105                 110

Tyr Gly Ala Ser Ala Val Thr Ser Asn Ala Thr Gly Gly Phe Glu Phe
        115                 120                 125

Thr His Arg Glu Ile Arg Lys Leu Trp Thr Glu Gly Pro Ala Arg Val
    130                 135                 140

Ser Val Tyr Glu Ser Phe Ala Trp Phe Tyr Ala Val Asn Thr Gly Gln
145                 150                 155                 160

Ile Ser Ile Arg His Gly Met Arg Gly Pro Gly Ala Val Val Val Ala
                165                 170                 175

Asp Gln Ala Gly Gly Leu Asp Ala Leu Gly Gln Ala Arg Arg Val Leu
            180                 185                 190

Arg Lys Gly Gly Val Leu Ala Val Ser Gly Gly Val Glu Ser Ala Leu
        195                 200                 205

Asp Pro Trp Gly Leu Ala Ala His Ala Ser Ser Gly Thr Leu Ser Arg
    210                 215                 220

Ser Gly Asp Pro Ala Thr Ala Tyr Leu Pro Phe Asp Arg Arg Ala Leu
225                 230                 235                 240

Gly Thr Val Val Gly Glu Gly Gly Ala Leu Leu Thr Leu Glu Thr Pro
                245                 250                 255

Arg His Ala Glu Glu Arg Asp Ala Pro Arg Ile Tyr Gly Glu Leu Ala
            260                 265                 270

Gly Tyr Ala Ala Thr Phe Asp Pro Pro Ala Gly Ser Gly Arg Pro Pro
        275                 280                 285

Gly Leu Glu Arg Ala Ala Arg Leu Ala Leu Ala Asp Ala Gly Leu Ala
    290                 295                 300

Pro Gly Asp Val Asp Val Val Phe Ala Asp Ala Ala Gly Leu Pro Ala
305                 310                 315                 320

Ala Asp Ala Ala Glu Ala Ala Ala Leu Arg Ala Leu Phe Gly Pro Gly
                325                 330                 335

Gly Val Pro Val Ser Val Pro Lys Thr Gln Thr Gly Arg Leu Ala Ser
            340                 345                 350

Gly Gly Pro Ala Leu Asp Val Ala Ala Ala Leu Leu Ala Leu Arg Asp
        355                 360                 365

Gly Leu Val Pro Pro Ala Val His Leu Asp Glu Val Asp Pro Ala Tyr
    370                 375                 380

Gly Leu Asp Leu Val Arg Asp Thr Pro Arg Ala Leu Pro Leu Arg Thr
385                 390                 395                 400

Ala Leu Val Leu Ala Arg Gly His Gly Gly Phe Asn Ala Ala Val Val
                405                 410                 415

Val Arg Gly Arg Arg Arg Pro Arg Thr Ala
            420                 425

(2) INFORMATION FOR SEQ ID NO:14:

(i) SEQUENCE CHARACTERISTICS:
(A) LENGTH: 83 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:14:

Met Ser Ala Leu Thr Val Asp Asp Leu Lys Lys Leu Leu Ala Glu Thr
1               5                   10                  15

Ala Gly Glu Asp Asp Ser Val Asp Leu Ala Gly Glu Leu Asp Thr Pro
            20                  25                  30

Phe Val Asp Leu Gly Tyr Asp Ser Leu Ala Leu Leu Glu Thr Ala Ala
        35                  40                  45

Val Leu Gln Gln Arg Tyr Gly Ile Ala Leu Thr Asp Glu Thr Val Gly
    50                  55                  60

Arg Leu Gly Thr Pro Arg Glu Leu Leu Asp Glu Val Asn Thr Thr Pro
65                  70                  75                  80

Ala Thr Ala

(2) INFORMATION FOR SEQ ID NO:15:

(i) SEQUENCE CHARACTERISTICS:
(A) LENGTH: 160 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:15:

Met Thr Thr Thr Thr Pro Pro Asp Asp Val Arg Ala Gly Ser Leu Pro
1               5                   10                  15

Gly Asp Ala Ala Arg Ser Ala Ala Leu Tyr Thr Glu Val Gln Ala Phe
            20                  25                  30

Tyr Ala Arg Gln Ala His His Leu Asp Ala Val Arg Ala Glu Glu Phe
        35                  40                  45

Ala Ala Thr Phe Ala Ala Glu Gly Val Phe Ala His Ser Pro Asp Thr
    50                  55                  60

Pro Ala Ala Arg Gly Arg Ala Ala Ile Ala Glu Glu Val Arg Gly Phe
65                  70                  75                  80

Asn Ala Arg Arg Phe Ala Asp Asp Pro Val Gln Arg Arg His Trp Phe
                85                  90                  95

Ser Met Leu Asp Val Arg Pro Gly Glu Asp Gly Ala Val Glu Thr Glu
            100                 105                 110

Phe Tyr Ala Leu Val Val Val Thr Arg Pro Asp Ala Ala Leu Pro Val
        115                 120                 125

Ile Gly Pro Ser Cys Val Val Arg Asp Val Leu Val Arg Glu Gly Gly
    130                 135                 140

Glu Leu Arg Thr Leu Ser Arg Gln Val Thr Gln Asp Arg Thr Leu Leu
145                 150                 155                 160

(2) INFORMATION FOR SEQ ID NO:16:

(i) SEQUENCE CHARACTERISTICS:
(A) LENGTH: 271 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:16:

Met Thr Thr Ala Ala Pro His Thr Arg Pro Gly Glu Ala Gly Thr Thr
1               5                   10                  15

Arg Gly Pro Ala Leu Val Thr Gly Ala Thr Arg Gly Ile Gly Leu Ala
            20                  25                  30

Val Ala Glu Ala Leu Val Ala Arg Gly Tyr Pro Val Val Val Cys Ala
        35                  40                  45

Arg Asp Ala Glu Ala Val Ala Arg Thr Val Lys Glu Leu Ala Ala Gly
    50                  55                  60

Gly Ala Arg Val Glu Gly Val Val Ala Asp Val Thr Asp Ala Ala Ser
65                  70                  75                  80

Val His Glu Leu Val Ala Thr Thr Val Ala Arg Phe Gly Pro Val Glu
                85                  90                  95

Val Leu Val Asn Asn Ala Gly Arg Ser Gly Gly Gly Val Thr Ala Glu
            100                 105                 110

Leu Ser Glu Ser Leu Trp Asp Asp Val Ile Ala Thr Asn Leu Lys Ser
        115                 120                 125

Val Phe Leu Val Thr Arg Glu Val Leu Thr Thr Gly Gly Met Thr Gly
    130                 135                 140

Arg Gly Arg Gly Val Val Asn Ile Ala Ser Thr Gly Gly Lys Gln Gly
145                 150                 155                 160

Val Val Phe Gly Ala Pro Tyr Ser Ala Ser Lys His Gly Val Val Gly
                165                 170                 175

Phe Thr Lys Ala Leu Gly Leu Glu Leu Ala Arg Ser Gly Ile Thr Val
            180                 185                 190

Asn Ala Val Cys Pro Gly Tyr Val Glu Thr Pro Met Ala Ala Gly Val
        195                 200                 205

Arg Arg His Tyr Ala Asp Leu Trp Asp Val Thr Glu Glu Asp Val Leu
    210                 215                 220

Ala Arg Phe Glu Ala Lys Ile Pro Leu Gly Arg Tyr Thr Arg Pro Asp
225                 230                 235                 240

Glu Val Ala Ala Leu Val Asp Tyr Leu Val Thr Asp Ala Ala Ala Ala
                245                 250                 255

Val Thr Ala Gln Ala Leu Asn Val Cys Gly Gly Leu Gly Asn Tyr
            260                 265                 270

(2) INFORMATION FOR SEQ ID NO:17:

(i) SEQUENCE CHARACTERISTICS:
(A) LENGTH: 331 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:17:

Met Thr Thr Thr Thr Thr Gly His Gln Arg Pro Gly Ser Ala Glu His
1               5                   10                  15

Ser Ala Arg Leu Ala Ala Pro Pro Ala Ser Ala Tyr Glu Leu Val Ala
            20                  25                  30

Asp Val Thr Arg Trp Pro Leu Leu Phe Thr Pro Cys Leu His Ala Glu
        35                  40                  45

Val Leu Glu Ser Gly Pro Gly Thr Glu Arg Val Arg Leu Trp Ala Leu
    50                  55                  60

Thr Gly Glu Gln Val Arg Gly Trp Thr Ser Arg Arg Thr Leu Asp Ser
65                  70                  75                  80

Glu Gly Leu Arg Val Gly Phe Arg Gln Glu Asp Ser Ala Pro Pro Leu
                85                  90                  95

Ala Ala Met Gly Gly Glu Trp Arg Phe Thr Glu Glu Gly Glu Asp Thr
            100                 105                 110

Arg Ala Val Leu Ala His Asp Trp Thr Leu Thr Glu Pro Gly Ala Ala
        115                 120                 125

Pro His Arg Trp Val Thr Glu Thr Leu Asp Arg Asn Ser Thr Ala Glu
    130                 135                 140

Ile Gly Ala Val Thr Ala Trp Ala Ala Arg Thr His Ala Ala Gly Gly
145                 150                 155                 160

Ala Asp Ala Leu Leu Phe Ser Phe Thr Asp Ser Leu Asp Ile Ala Ala
                165                 170                 175

Pro Ala Pro Asp Val Tyr Ala Phe Leu Asp Ala Ala Asp Gln Trp Pro
            180                 185                 190

Ala Arg Leu Pro His Val Ser Arg Val Ala Phe Ser Thr Thr Pro Ala
        195                 200                 205

Thr Pro Leu Thr Ala Gly Ala Glu Val Gln His Leu Glu Met Glu Thr
    210                 215                 220

Arg Ala Asp Asp Gly Thr Arg His Leu Thr Arg Ser Ile Arg Leu Gly
225                 230                 235                 240

Phe Ala Gly Arg Leu Leu Val Tyr Lys Gln Thr Thr Leu Pro Ala Pro
                245                 250                 255

Leu Leu Gly His Ala Gly Ser Trp Ala Leu Glu Pro Leu Pro Gly Gly
            260                 265                 270

Gly Thr Arg Val Thr Ala Arg His Arg Val Ala Leu Asp Pro Asp Ala
        275                 280                 285

Val Thr Glu Arg Phe Gly Ala Gly Thr Thr Leu Ala Ala Ala Arg Asp
    290                 295                 300

Thr Val Arg Ala Leu Leu Gly Gly Asn Ser Arg Arg Thr Leu Glu Ala
305                 310                 315                 320

Ala Arg Ala His Thr Glu Ser Ala Gly Glu Arg
                325                 330

(2) INFORMATION FOR SEQ ID NO:18:

    (i) SEQUENCE CHARACTERISTICS:
        (A) LENGTH: 314 amino acids
        (B) TYPE: amino acid
        (C) STRANDEDNESS: single
        (D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:18:

Met Thr Thr Thr Gly Pro Ala Thr Pro Leu Ala Pro Gly Pro Ala Ser
1               5                   10                  15

Ala Glu Thr Val Ala Leu Ala Asp Gly Val His Ser Trp Leu Gln Pro
            20                  25                  30

Asp Gly Gly Trp Cys Val Ser Asn Ala Gly Ile Leu Leu Ala Pro Asp
        35                  40                  45

Arg Val Ala Leu Val Asp Thr Ala Ala Thr Glu Ala Arg Ser Arg Ala
    50                  55                  60

Leu Gly Ala Ala Val Ala Gly Leu Ser Pro His Pro Val Arg Leu Leu
65                  70                  75                  80

Val Asn Thr His Phe His Gly Asp His Ser Phe Gly Asn Gly Ile Leu
                85                  90                  95

Gly Lys Asp Ala Val Ile Val Ala His Glu Arg Thr Arg Thr Glu Met
            100                 105                 110

Ala Glu Ala Gly Leu Gly Leu Thr Gly Leu Trp Pro Gly Val Asp Trp
        115                 120                 125

Gly His Val Asp Pro Val Leu Pro Gln Leu Thr Tyr Arg Arg Arg Leu
    130                 135                 140

Thr Leu His His Gly Asp Leu Arg Val Glu Leu Ile His Pro Gly Pro
145                 150                 155                 160

Ala His Thr Thr Asn Asp Thr Leu Val Trp Leu Pro Glu Gln Arg Val
                165                 170                 175

Leu Phe Ala Gly Asp Val Leu Leu Pro Gly Ala Thr Pro Phe Val Leu
            180                 185                 190

Met Gly Ser Val Thr Gly Ser Leu His Thr Leu Arg Leu Leu Arg Arg
        195                 200                 205

Leu Gly Pro Arg Val Val Val Gly Gly His Gly Pro Leu Ala Gly Pro
    210                 215                 220

Glu Val Ile Glu Glu Thr Glu Arg Tyr Leu Leu Arg Leu Arg Arg Ile
225                 230                 235                 240

Ala Thr Glu Gly His Ala Ala Gly Leu Thr Pro Leu Glu Ala Ala Arg
                245                 250                 255

Arg His Gly Pro Gly Pro Phe Ala His Trp Ser Glu Pro Glu Arg Leu
            260                 265                 270

Ala Ala Asn Leu His Arg Ala Tyr Ala Glu Leu Gly Pro Ala Pro Leu
        275                 280                 285

Gly Thr Pro Leu Asp Val Leu Ala Cys Phe Gly Asp Leu Ile Ala Tyr
    290                 295                 300

Asn Asp Gly Glu Leu Pro Val Ser His Ala
305                 310
(2) INFORMATION FOR SEQ ID NO:19:

    (i) SEQUENCE CHARACTERISTICS:
        (A) LENGTH: 305 amino acids
        (B) TYPE: amino acid
        (C) STRANDEDNESS: single
        (D) TOPOLOGY: linear

    (ii) MOLECULE TYPE: protein

    (iii) HYPOTHETICAL: NO

    (iv) ANTI-SENSE: NO

    (xi) SEQUENCE DESCRIPTION: SEQ ID NO:19:

Met Thr Gly Arg Val Thr Gly Thr Met Ala Gly Pro Leu Pro Gly Thr
1               5                   10                  15

Val Leu Val Ala Gly Ala Ser Gly Ala Val Gly Gly Arg Leu Val Ser
            20                  25                  30

Arg Leu Leu Asp Ser Gly Val Pro Val Arg Ala Leu Val Arg Ser Ala
        35                  40                  45

Ala Arg Gly Arg Ala Leu Ala Ala Val Gly Ala Glu Thr Val Val Gly
    50                  55                  60

Asp Leu Ala Asp Thr Ala Ser Leu Ala Ala Ala Leu His Gly Val Glu
65                  70                  75                  80

Arg Ala Phe Leu Leu Leu Gln Asp Glu Ala Gly Ala Pro Phe Ala Arg
                85                  90                  95

Ala Ala Ala Lys Ala Arg Gly Leu Arg Glu Val Val Val Leu Ser Ala
            100                 105                 110

Thr Ala Ala Ala His Pro Glu Tyr Asp Asn Pro Met Phe Gln Lys His
        115                 120                 125

Val Arg Gly Glu Arg His Val Arg Gly Ser Gly Val Pro Trp Val Phe
    130                 135                 140

Leu Arg Pro Gly Ala Phe Ala Ser Leu Ala Leu Leu Trp Ala Pro Ala
145                 150                 155                 160

Leu His Gly Asp Gly Val Val Arg Val Pro His Ala Gly Leu Ala Val
                165                 170                 175

Pro Leu Ile Asp Pro Arg Asp Ile Ala Asp Val Ala Ala Ala Val Leu
            180                 185                 190

Thr Ala Pro Val Glu Arg Trp Ala Gly Lys Ala Val Pro Leu Ser Gly
        195                 200                 205

Pro Glu Val Leu Thr Leu Pro Gly Arg Thr Ala Val Leu Ala Ala Glu
    210                 215                 220

Leu Gly Arg Pro Leu Arg Val Glu Pro Val Pro Glu Glu Glu Trp Val
225                 230                 235                 240

Gln Leu Thr Ala Arg His Ile Pro Glu Pro Tyr Ala Arg Ala Leu Ala
                245                 250                 255

Gly Val Glu Arg Phe Phe Thr Glu His Pro Pro Ala Val Ser Pro Gly
            260                 265                 270

Val Thr Asp Val Thr Gly Arg Pro Ala Arg Ser Phe Ala Thr Trp Val
        275                 280                 285

Arg Asp His Ala Ala Ser Phe Ala Pro Gly Pro Pro Thr Val Val Gly
    290                 295                 300

Gly
305

(2) INFORMATION FOR SEQ ID NO:20:

    (i) SEQUENCE CHARACTERISTICS:
        (A) LENGTH: 396 amino acids
        (B) TYPE: amino acid
        (C) STRANDEDNESS: single
        (D) TOPOLOGY: linear

    (ii) MOLECULE TYPE: protein

    (iii) HYPOTHETICAL: NO

    (iv) ANTI-SENSE: NO

    (xi) SEQUENCE DESCRIPTION: SEQ ID NO:20:

Met Thr Glu Gln Ser Thr Thr Leu Ala Ala Pro Ala His Ala Glu Arg
1               5                   10                  15

Thr Gly Arg Arg Thr Lys Gly Pro Gly Arg Pro Pro Asp Arg Ser Arg
            20                  25                  30

Asn Gly Pro Gly Arg Ser Pro Arg Ser Arg Pro Gly Thr Arg Ala Ala
        35                  40                  45

Val Arg Arg Ser Gly Val Ser Pro Gly Lys Ser Ser Ala Gly Ile Val
    50                  55                  60

Ala Ala Gly Phe Ala Arg His Phe Val Pro Ala Ala His Gly Gly Ala
65                  70                  75                  80

Glu Gly Gly Phe Gly Glu Leu Ala Glu Ala Val Leu Arg Leu Gly Thr
                85                  90                  95

Gly Cys Thr Ser Ala Ala Trp Ala Ala Ser Leu Ser Ala Tyr Ala Gly
                100                 105                 110

Arg Tyr Ala Ala Tyr Leu Pro Glu Glu Gly Gln Ala Glu Val Trp Ala
        115                 120                 125

Glu Gly Pro Asp Ala Leu Leu Ala Gly Ala Leu Val Pro Ser Gly Thr
    130                 135                 140

Val Thr Pro Val Pro Gly Gly Trp Arg Leu Asp Gly Ala Trp Pro Tyr
145                 150                 155                 160

Ile Ser Gly Val Arg His Ala Ala Trp Val Leu Ala Cys Ala Thr Val
                165                 170                 175

Pro Gly Gly Glu Gly Glu Glu Gly Pro Glu Val Arg Phe Phe Ala Gly
                180                 185                 190

Pro Arg Ala Ala Pro Arg Val Glu Arg Thr Trp Asn Thr Thr Gly Met
        195                 200                 205

Arg Ala Thr Gly Ser Asp Thr Leu Val Leu Asp Asp Val Leu Val Pro
    210                 215                 220

Ala His Arg Ser Phe Pro Arg Thr Arg Val Leu Ala Gly Gln Arg Pro
225                 230                 235                 240

Arg Val Ala Gly Ala Val Pro Thr Val Arg Met Ala Arg Val Gly Ala
                245                 250                 255

Leu Pro Val Val Thr Pro Leu Val Gly Ala Ala Arg Gly Ala Leu Arg
                260                 265                 270

Ala Trp Thr Glu Arg Ala Ala Gln Gly Arg Ala Pro Ser Pro Gly Ala
        275                 280                 285

Leu Gly Glu Leu Ser Arg Ala Ala Gly Glu Ala Asp Ala Ala Glu Leu
    290                 295                 300

Leu Val Leu Arg Ala Ala Ala Ala Ala Asp Gly Thr Val Ser Leu Pro
305                 310                 315                 320

Glu Pro Ala Ala Ala Val Arg Gly Lys Arg Asp Thr Ala Leu Ala Ala
                325                 330                 335

Glu Leu Ala Val Gly Ala Val Gln Arg Leu Val Arg Ala Ser Gly Thr
                340                 345                 350

Ser Gly Gln Ser Pro His Asp Pro Val Gln Arg Phe Trp Arg Asp Val
        355                 360                 365

Gln Thr Gly Ala Ser His Val Ala Leu Ser Pro Glu Ala Ala Gly Ala
    370                 375                 380

Ala Tyr Gly Ala Trp Ala Val Gln Glu Ala Asn Arg
385                 390                 395

(2) INFORMATION FOR SEQ ID NO:21:

    (i) SEQUENCE CHARACTERISTICS:
        (A) LENGTH: 108 amino acids
        (B) TYPE: amino acid

(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO:21:

Met Ser Gly Gly His Met Val Thr Met Ile Asn Gln Met Leu Leu
1               5                   10                  15

His Gly Asp Glu Ser Arg Phe Leu Ala Val Leu Glu Glu Ile Cys
                20                  25                  30

Ala His Met Arg Ala Gln Pro Gly Phe Leu Ser Leu Arg Leu His
                35                  40                  45

Arg Ser Pro Asp His Pro Glu Arg Trp Ala Met Leu Ala Asp Trp
                50                  55                  60

Ser Asp Ala Ala Ala His Arg Ala Ala Ala Ser Ala Pro Gly Ile
                65                  70                  75

Arg Pro Ala Phe Ala Arg Leu Arg Ala Glu Ala His Thr Ala Pro
                80                  85                  90

Gln Val Tyr Ala Pro Val Pro Thr Pro Gly Ala Pro Ala Gly Asp
                95                  100                 105
Ser Leu Ala

(2) INFORMATION FOR SEQ ID NO:22:

(i) SEQUENCE CHARACTERISTICS:
(A) LENGTH: 24379 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:22:

CAATTGCCAT GGAATGCCCG GTTATAAGGG AAGTGGAATG TGTCACCGCC GTCGTCGCGT
60

GGGGCGGTGG GCGGCTAATG TGAGCGATCC CGGTGGCCGC GACCGTACTC ATCGCGGCGC
120

GTCACCGATT GACCCGAGTG CCTCCCGCCG CGTCTCCACC CCACCTGGAG GGCGGGTCGG
180

CGCCGCGTGC CCCGTGACGG AGGATTTGGA CAACCGTGGA CGCGCAAGGC CGTGGGCGGG
240

GGGAAGAATT CGACCCCGCA GACCAATGGG TACTCAATCC GCAGACCGGC GAATACGAAC
300

TGCGACTCGG CGGCTCCGCT GAGCAGTCGC AGGAGCCGCC GGGGTCCGGC GGCGGGGCCT
360

GGGACCACGT CGACGGGTCC GCGCGTCCCC GCAGAACCGA GCCGTACCGC TCCGAGCCCC
420

GGGTGTCCGA GGCAGCGGCG GCTCCCCGCG CACGGAGAAC GCCCCGCGCG CATGGCGGTC
480

GCGCGGCGGC GCAGTAGGAG GCGGCGGCCG GCGGTCGTCC CTCGGCCCGC AAACAGGCTT
540

CCGGGCGGGC CGCCTCGCGC AAGGCGCGCA AGGGCGGCGG CAAGAAGAAG GCGCTGCTGT
600

GGACGGCCGG GGGGCTCGGT TTCGCGCTCG TCGCGGTGGC CGGCAGCGGC TACGCGTACT
660

ACCAGCACCT CAGCGGCAAC ATCGACAAGA TTGACGTCGG CGACGCGGGC AACAAGGACG
720

CCGCCCCCGA CGGGCCGATC AACATCCTGA TCATCGGCAC CGACAAGCGC ACCGGCAAGG
780

GCAACGAGGG GTGCGGGGGC AAGGACAGCC CTGGCCACGC GGACACCAAC ATCTTGCTGC
840

GCGTCTCCGC CGACCGCACC AACACGACCG GCCTGAGCAT TCCGCGTGAC CTCATCACGA
900

ACATCCCGGA CTGCCTGACC ACCCAGGACG ACGGTTCGAA GAAGACGATC CCCGGCACGC
960

AGAACGTCCG CTTCAACACG AGCCTCGGGC AGGAGGGCCG CGACCCCGGC TGCACGATGC
1020

GCACGGTCAC CGAGCTGACC GGGCTCAAGG TCGACCACTT CATGATGGCC GACTTCAACG
1080

CGGTCAAGAA CCTGACGACG GCCGTGAACG GCGTCGAGGT GTGCGTGGCG AAGGACGTCG
1140

ACGACCCCGA CTCGCACCTC AAGCTCTCGG CGGGGACGCA CAAGGTGCAG GGCGAGCAGG
1200

CGCTCGCCTT CGTCCGCACC CGGCACAGCT TCGGCAACCA GGGCGACCTC GACCGCATCA
1260

AGGTCCAGCA GCAGTTCCTC GGTTCGCTCG CGCGGCAGCT CAAGTCCGAG GACACGCTGA
1320

CGAGTCCGAA GAAGCTCTAC AAGGTCGCCG AGGCGGCGAC CGACGCGCTG ACCGTGGACT
1380

CGGGGATCGG CAGCATCACG AAACTGATGT CGCTGGCCAA GGAGTTGCAG CACATCAATC
1440

CGAAGAACAT CACCTTCGTG ACGCTCCCCG TGGTCGACAA CCCGGCCGAG AAGGTCAAGG
1500

CGACGGTCGT CCTCAATGAG ACGGACGCTG ATCCGCAGCA GTCGGCTCTC GGGCAGAGTC
1560

TCGACGTGGG CCGCCAGCTC GTCGACTCGC TCACCGACCA GGACCCGCGC GACGGCAAGA
1620

CCGTCCCGTG GCTCGCGACG CGCTGGAAGG CCGACCCCGA GGCCACCCGC TTCACCTTCA
1680

CCCTGCGCGC GGGCGCCACT TTCAGCGACG GCACACCGGT CGACGCGCGC GCGGTCAAGG
1740

CCAACTTCGA CGCCGTGCAC GAGCTCGGCG CCGCCGCCTC GCGGGGGGCC GTGTACCTCG
1800

ACGGCTACCG CGAGACGCGC GTCAGCGGCG CCCGTACGCT CACGGTCGTC TTCGACAAGC
1860

CGAACGCCCA GTTCCTGCGC GGCACGTCCA CCGTCTCACT GGGCCTGCTC TCGCCGGGCA
1920

GCCTGCGCCG CACCCCGCAG GAGCGCTGCA CCGGGCGCCT CGTCGGCTCC GGTCCCTTCG
1980

TGCTCGACCG CTACCGGCCG AACACCTCCG TCACGCTGGA CCGGAGAAAG GGCTACTCCT
2040

GGGGCTCGCG CCTGTGGCAA CGGGAGGGCG GCGCGTACCT GGAGGGGGTC GAGTACCGGA
2100

TCGTGCCCGA GAACACGACC CGCTCGGGCG CCCTGTCCGC CGGTCAGCTC GACGTCGCCA
2160

CCGCGCTCGC GCCGCAGGAC AGGGAGCGGT TCTCCGCCCC CGGCTGGTCG CTGCTGACCC
2220

GTACCGCGCC CGGCGTCGAC CTCAGCCTGT ACGTCAACGC GCGCCGCACG GCGCTGCGCG
2280

AGGCGGCGGT GCGCCAGGCC CTCCAGAAGG GCATCGACCG CGAAGCCGTC GCCACGACCT
2340

TCCTCAGTTC CCGCAAGCTC GCGGCGACCA GCGTGCTCTC CTCCACGACC CCCGGCTACA
2400

CCGACCTCGG CGACCGCCTC GCCCACGACC CTGCGGGTGC GAGGCGGCTG CTCGACAAGG
2460

CCGGATGGCG GCCCGGGGCG GACGGCATCA GGGTCAAGAA CGGCGTCAGG CTGCGACTCG
2520

ACGCCGTCTT CGTCCGGCAG CAGAGCCTCG AACTCGTGCA GCAGCAGCTC AAGGACATCG
2580

GCGTCGAACT GCGGCTGAGA CAGCTCACCG TCTCCCGTTT CCCCGAAGTG CTCGCCGCGG
2640

GCAGCTACGA CCTGAGCCTC CAGAGCGCCA ACCGCGCCGA TCCCGACGTC CTCACCACGG
2700

CCTTCGCGGG CGGCACCCCC GTCGCCGACG CGCGCCTGCG CTCCGAACTG CGCCGGGCCA
2760

CCTCCTCCAC CGACGAGGCG ACGCGCTCCT CGCTCTTCGC CGCGGCGCAA CGGCGTCTCG
2820

TCGACGAGGG GCACGTCCTG CCGCTCAACG AGACCGAGGA GACCGCCGCG CTCTCGACGC
2880

GGGTGCACGG GCTGACGCGG GACGCCTCCA ACCGGCTGGT CCTGCACGAC ACCTGGACCA
2940

CCGGGTGACG GCCCGCTACC TGGCCCGCCG GCTGGGCCGC GTCGTCCTCG TCGTCTGGGC
3000

CGCGTACACC CTGTCCTTCG CCGTCCTCTA CCTGCTGCCG GGAGATCCCG TGCAGACCAT
3060

GCTGAGCGGC GCGGCGGGCG GGGACGGGGC GGCCGTCGAC CCGCACGAGG CCCAGCGGCT
3120

GCGCCACACG CTCGGCCTCG ACCGCCCGCT CGCCGTCCAG TACACCAGCA TGCTGGGGCA
3180

CGCCCTGCGC GGAGACCTCG GCACCTCGAT CCGCAGCGGG GCGCCCGTAC GCGGGCAACT
3240

CGCCCAAGCG CTGCCCGACA CCCTCTCGGT GGCCCTCCCC GCGCTCGTCC TCAGCGTGCT
3300

CGTCGCGCTC TGCCTCGCCC TCCTCGGGGC CTGGCCCCGG CGACGAGCCC TGCGCAGGGC
3360

GGCGACCGCC CTGCCCTCGC TCGGCACCGC GATGCCCAGC TTCTGGCTGG GGCTGCTCCT
3420

CGCCCAGTGG GTCTCCTTCC GCTGGGGACT GCTCCCCGCC ACGGGCGGCG GGCGCTCGCC
3480

CCGGGCCACC CTGCTGGCCG CGCTGACCCT CGCCCTGCCC ATCGGCTGCG TGCTCGCCCA
3540

GGTGCTGGGG AGGGGCCTGC GCGCCGCGCT CGCCGAGCCG TACGCGGACG TGGCGCGCTC
3600

GCGCGGCGCG GGGCGGGCCA GGCTGCTGCT CGCGCGCGCG CTGCGCAACG CCTCGGTGGC
3660

CGCCCTGGCC CTGCTCGGCG TCGTGTGCGG GCAACTCCTC GCCGGGGCCG TCCTGGTGGA
3720

GACCGTCTTC GCCAGGGGCG GCATCGGGCG CCTCGCCATG GACGCCGTCA CCTACCAGGA
3780

CCTGCCCGTC GTGCAGGGCG TGGTGGTGCT CGCCGCCCTC GTCGCGGCGC TGGTCAACCT
3840

CGTGGTGGAC CTCCTCCTCC CGCTCCTCGA GCCGCGTACC GCCTCGGAGG CCGCCGATGC
3900

CGTCCCTGCC CACTGACTCC CCGGGCCCCG CCCCGTACCC GGCCCCTCCC GCCCAGCCGG
3960

TCACGGACCG CGCCGAGGGC CCGGAGCCCC GGGAGGCGGG AGCGCGCCGC GCGGGCCGCG
4020

TGGCGCTCCG GCGCGTCGCC GCGCTGTGGC GGGCGCCCGG ACTGGCCCTG TCCCTGCTCG
4080

TCCTCCTGCT CGTCCTCGGC TGGGCGCTGC TGCCCGGCCT GTTCACGGCG GCCGACCCTC
4140

TGCGGGCCGA CGCCGCGCAC CGCCTGCTCG CCCCCGGCGC CGGACACCCG CTCGGCGCCG
4200

ATCACGTCGG CCGCGACCTC TACGCGCGGG TCGTGCACGG CACCGCGCGC TCGCTGGGCA
4260

CGGCGTTCGC CGCCGTCGCG CTCGGGGTGC TCGCCGGCGG TGCGCTCGGG GCCGTGGCCG
4320

GAGTGGCGGG GCGCGCGGTG GACGCCGTCG TCATGCGCGT CGTGGACGTC CTCCTGGCCG
4380

TCCCCGGGCT GCTGCTCTCG CTCGCGGTGG TCTCCGCGCT CGGCTTCGGC ACCGCGCAGG
4440

TCGCCTGCGC GGTCGGCGTG GGCACCGTCG GCGGGATCGC CAGGGTGAGC CGTGCGCAGG
4500

TGCGGCGCGT GCGCGGCGGC GAGTACGTCG AGGCCGCGCG CCTCGCCGGG GTCGCGGGGC
4560

CGCTGATCCT GCTGCGCCAC ATCGTGCCCA ACGCGGCTCC GCCCGTGCTC GCGCTCGCCG
4620

TCACCGAGTG CGGCACCGCG GTACTCGGCG TCGCGTCCCT CGGCTTCCTC GGCTTCGGCG
4680

CGCCGCCGCC CGCCCCGGAG TGGGGCGCCC TCATCTCCAC CGGCCGGGAC TACCTCGTCT
4740

CCGCCTGGTG GCTCACCACC CTGCCGGGCC TCGTGCTCGT CGCCCTCGTC GTCGCCCTGC
4800

ACCGCGTCGG CCGCGCCCTG GAACGAGAGG AACGCACCGG ATGACCCCGG CGGACAAGCC
4860

GACCGACGAG CGCTCACCTG TCCTGGACCT CAGTGGTGTC AGCGTCGCCT ACGGGACGCG
4920

CACCGTGCTG CACGGCATCG ATCTGCGCCT CGCGCCGGGG CAGGTGCTCG CGGTGCTCGG
4980

CGCCTCGGGT TCCGGCAAGA GCACCCTCGC GCAGGCGGCT CTCGGCCTCC TGCCCCCCGG
5040

CGGCCGGGTG ACGGCAGGGC GCGTCACGGT CGCCGGGCAC GACATCACCG CGCTCGCGCC
5100

GCACCGGCTG CGCGCGCTGC GCGGCACGGT CACCGGACTC GTGCCGCAGG ACCAGGCGGT
5160

CTCCCTCGAC CCCCTCGTCC GCGTCGGCGC GCAGGTCACC GAGACGCTGC GCGCCCATCG
5220

CCTGGAGGAC CGCCGCGAGG CCGCCCGGCG CGCCGTGCCC TTGCTCGGCG AGGCGGGGAT
5280

CGAGGCCCCC GGGCCGCTCG CCCGCGCCTA CCCGCACGCG CTCTCCGGCG GCCAACGCCA
5340

ACGCGTCCTC GTCGCCGGGG CCTTCGCGGC CCGCCCCCCG CTGGTCGTCG CCGACGAGCC
5400

GACGAGCGCG CTCGACGCGA CCGTGCGGCG CCGGGTCATG GACCGCTTCG CCGCCCTCGT
5460

CGCCGCGCAC GGCACGGCCG TCCTCCTCGT CACCCACGAC TTCCGGCTCG CGAGGGAACG
5520

CGCCGACCAG GTCGCCGTCC TCGCGGACGG CCGTCTCGTG GAGAGCGGCC CCGCCGCACG
5580

GGTCCTCGAC CGGCCCGCCC ACCCGTACAC CCGCCGCCTC ACGGGGGCCG GCCGCCGCGT
5640

GGCCGCACGG GGCACGGCGC CGCGCGCGTC GGGCACCCCC GTGGTGCGCG CCCGCGACCT
5700

CGTCAAGGAG TACCGGCGGG ACGGGCGGCG GGTGCGCGCC GTGGACGGGG TCGGCTTCAC
5760

GGTCCGCGAA GGCGAGTTCT TCGCCCTCGT CGGCGAGTCG GGCTCGGGCA AGTCCACGAC
5820

CGCCCGGCTC GTGACGGGTC TGACCGCGCC CACGTCGGGC GCGGTGGAGC ACGCGCCCGC
5880

TCCGGTGCGG CCCCAGCTCG TCCAGCAGAG CCCGTACGCG GCCTTCGACC CGCGCTGGAC
5940

CGTGCGCCGC ATCGTGGAGG AGCCGCTGCG GGCGCGGCAC GTGCCCGGGG CGCGGCGGCG
6000

GGCGCGGCTG CGCGAACTGC TCGCGCTCGT CGGCCTCGAC GAGGAGCTGC TCGCGCGGCG
6060

GCCCCGGGAG CTGTCCGGCG GGCAGCGGCA GCGGGTGGCG CTCGCCCGCG CGCTCGCCCC
6120

GGAACCGCGC CTGCTCGTGT GCGGCGAACC CGTCGCCGCC CTCGACCCCG TCGCGCGCGA
6180

GCGCGTCGTC CACCTCCTGG AGCGGCTGCG GGCGGAGCTG GGCCTCACGT GCCTCTTCGT
6240

CTCCCACGAG CTGGACGTCG TGCGGCGGTT GTGCGGTCGC GTCGCCGTCA TGCGGGGCGG
6300

GCGGCTACTG GAGAGCGGAC CGGTGGGGGA GGTGCTGTCG GCGCCCTCGC AGCCGTACAC
6360

GCGGGCGCTG CTCGCCGCGG AGGCGGGCCC TTCCGACACG CCCGGAGCGG GGTGAGCTGC
6420

GTCACGTCGG GAGGGGCTTG CGCGGCCCCC GCCAGCCCGC CTACGCGAAG GGAGGGGGCT
6480

TACCTGGAGG TATGGGCTTA CCCGGGGAAA GTCTGCAGCC TAGGCTCGCC CCGTGAACGA
6540

GATCACTGTC GAGATCTGGA CCGACGTCGT CTGCCCGTGG TGCTACATCG GCAAGCGGCG
6600

CTTCGAGCGG GCGCTGGCCG CCTTCGACGC GAAGGAGGAC GTGCGCGTCC ACTGGCGCAG
6660

CTTCGAACTC GACCCCGCCG CCCTGCGGGT CACCGACGAG ACCATCCCCG AGCGCATGCT
6720

GCGCCGCCAG GGCATCCCGC CCGAGCAGGC CGCCGAACTC CTCGCCGGGG TGAGCGCCCA
6780

GGCCGAGGCC GAAGGGCTGG AGTACCACCT CGACCGCGCC CGGCCCTGCA ACACCTTCGA
6840

CGCGCACCGG CTCGCCCACC ACGCGGGCAC GCGGGGCCTC GCGGAGACCT TCCAGGAACG
6900

GCTGATGTGC GCCTACACCG CCGAGGGCGT CTCGGTGGGC GACCACCCCA CCCTGCTGGC
6960

CCTCGCCGAG GAGGCGGGGC TCGACGCGGC CGCCGCCGCC GAGGTGCTGG CCGGTGACGC
7020

GCACGCCGAG GACGTACGGG CCGACGAGGA CCGGGCCGCC CGGCTCGGCG TCGGAGGGGT
7080

GCCCGCCTTC GTGATCGGCG GACGCTGGTC CGTCTCGGGC GCCCAGCCCG CCGAACTCCT
7140

CACCGGTCTC CTGGAACGGG CCCGGACCGC AGCCGCCGCC TGACGGTCAC CCGGACCGTC
7200

CGCGGGGCGG ACGCGGCCGG GTACCGCGCC GCAGTCTCCG CACCCCGCTT CACCCCTCCA
7260

CGCCCGCACC GGTCCCGTCC GCCCCCGGGC CGTCCCGCCC GGGGGCCGGT CGCCCCTTCC
7320

GCCGTCCCAA GGAAAGCGTC GCCATGTCCT CCTCCCCGCC CGCCCCCGCC ACCCCGGGCG
7380

TTGCCCCGCA CTCGCCGCCC GCTCCGCGCC TCGGCCTCGT GCTCCTCGTG TGCTGCCTGG
7440

CGCAGTTCCT CGTGACGCTC AGCGTCGCCA TCGTCAACGT GGCGCTCCCC GACATCCAGC
7500

GCGGGCTCGG CTTCAGCGCC GAGAGCCTCC AGTGGGTCGT CAACGCCTAC ACCGTCACCT
7560

TCGCGGGCTT CCTGCTCCTC GGCGGACGGA TCGCCGACCT CTTCGGGCGG CGCCGCATCT
7620

TCCTCGCGGG TGTCGCGCTC TTCGCCCTCG CGAGCCTCGC CGGGGGCCTA AGCCAGAACG
7680

CGGGGACCCT CGTCGCCGCG CGCGCCGTGC AGGGCCTCGC CGCCGCCGTC ATCGCGCCCA
7740

CCACCCTCGC CGTGCTCGGC ACGAGCTTCA AGGACCCGCA CCAGCGCCAC CGCGCCTTCG
7800

GCGCCTGGGG CGCGGTCTCC GGTGCGGGCG GCGCCTTCGG CGCCCTCGCG GGCGGCGCGC
7860

TCACGGACGC GTTCTCCTGG CGCTGGGTGC TCTTCGTCAA CCTCCCGATC GGCGTGCTCC
7920

TGCTCGCCGG GATCGCCTGG GGCATCAGCG AGTTGCGGCA CGCGGGCGAG GACCGCCGCA
7980

TCGACGTCGC GGGCGCCCTC ACCGTGACGC TCGGTCTCCT CGCGCTCGTC CTCGGCATCG
8040

TGCAGAGCGG CCCGCACGGC TGGGGCTCCG CCGCCACCCT CGTCCCGCTC CTCGGCGGCC
8100

TCGCCCTGCT CGGTGCCTTC GTTCTCGTCG AGGGGCGGTT CGCGCCGCAG CCGCTCATAC
8160

CGCTCGGCAT CTTCCGCTCC CGCTCCGTCG TCGCGGCCAA CGTCGTGGCG ATGACCAGCG
8220

GCGCCGCGCT CTTCAGCATG TTCTACTTCC TCACCCTCTT CCTCAACCAG GTACGGGACT
8280

ACAGCCCGCT GCGCACCGGC TTCGCCTATC TGCCGCTCGC GCTCGCCATC ATGGTCGCCG
8340

CGCAGTTCTC CGCCGCGCTC GTGCGCGTCC TCGGCCCCCG TACGACCCTG CTCGTGTCCA
8400

TGGCTCTCAC CGCCGCCGGA CTGCTCTGGC TCTCGCGGCT CACCGAGGAC TCCGGGTTCG
8460

CGGGCGGACT CCTCGGCCCG ACCCTGGTCG TCGGCATCGG ACAGGGCATC TCGATGTCCG
8520

CCTCCGCGAT CGCGGGGGTC GCCGGGGTGC GCCCGCAGCA GGCCGGGCTC GCCTCCGGGC
8580

TGCTCAACGC GACCCGGCAG CTCGGCGGCG CCCTCGGCCT CGCGGTCGTC GCCGCCGTCG
8640

CCACCTCGCG CGCCGACGGA CTCCTCGACG GGGTCGCGCG GCCCACGGCG GAACTCGCCC
8700

GGCACGCCCA GGCGTCCGGG CACCCGCTCT CCATCGCCGT GGCCGCGGCG CTGTCCGCCG
8760

TCGGCCTGCT CGCCTCGCTC GCCGCCCCGG GCCGCTCTCC GGCCCCCACC GGCACCCGCA
8820

CGGGCGGCGA CTCCGCCGCA CCGGCTCCCG CGGCGGCCCC CGCCGCCACC GGGACCACCG
8880

GCCCCGGCGA GATCTGAGCG CTCCCCCCGG TGACCGCCCG CTCCGGCGGG GCGGTCACCG
8940

GGGACGGCGC GGCCTGCGTA TAAACATGCG CGGGCCTTTT GCATGTGTGG GAAAGATGCG
9000

GGGCGTGCAC ATAATCACCG GCTTGCGCGC CGCGACTGTG TGCTGCGTCA CGTTTCACGT
9060

GGTGTCTATA TTTCCCTGTC TATTCTTGGC ACCGCTCGGA GGGGGACCGG TGCGCGCTGC
9120

CGTTTATCCC GCCGGACGTC ATGATGTCCA CCGGGAGGGA AACGTGGAAA TCAAGTACAT
9180

GGGTCAGTTG ACCATGCGGT GGGAGGGGCG GGAAAAGCTC CCCTCGGCGC GCAAGCCGCG
9240

CACCGTGCTC GCCCTTCTGC TCCTCAACGA CAAGACACCC GTCACGACCA GCGCACTCAT
9300

CACGGAACTC TGGGGCGAGA ACCCGCCGCG CAGCGCCCTG ACCACCCTCC AGACCTATAT
9360

TCTCCAGCTC CGGAAATGCC TCGCGGCGAT GAGCGGGCGA AGCCTCGCCT GCATATCCGA
9420

GAAAACCCTG GTGACGTGGC CCTGCGGTTA TCTGGCGCGG CTTCCGGCGG ACGCCACTTC
9480

GGATGTGGCT GAATTCCGTC GGTTTGCCAG GGAGGGGCGG GAAGCGGAAC GCAGGGGTCA
9540

TCTTGCCGAG GCCGTCCGCT CCTACCGCGC GGCGCTGTCA CTGAGCCAGG GACCACTCCT
9600

CGCCGATATC GAGCACGGGC CGCTGCTGCG CGCCGAGGCG GTACGCATGG AGGAGTGCCG
9660

GCTTTCGCTC GTCGAACGCT CCATCGAGGG CGACCTGCTC CTCGGCCGGC ACCGGGAGGT
9720

CGTCAGCGAA CTGTCCGCCC TCGTCGCCCA GTACCCCTAC CACGAACAGC TCACGGGGCA
9780

GCTCATGGTC GCGCTCGTGC GCTGTGGCCG CCGGCAGGAC GCGCTCGCCG TGCACCAACG
9840

GCTCCGGGCC CGCATGGTGG AGGACCTCGG CCTCGAACCG AGCAGCCACC TGCGCGCCCT
9900

GCAGTCCGCC GTCCTCAGCG GCGAGCCGCT GCCGGGACCG CCCGGAACCG GTGGCGAGAT
9960

CCCCACCCCC TACGCGGGCG CTTTCGCCAC CGCGGCGCGC TCGCACGACT GACCCCGCCC
10020

GGGGCCCGGT CCACCCGTCG CCGTCGCCGG GTGGAAGGGC TGGACGGAGA ACAGGCCCTC
10080

TCCTTCCCGC CGCCGGGGCC CGCGCTCAGC GCAGCGCGCG AAACCCCCGC TCCCAGTGCA
10140

CGAGCGGTTC GCCCTCACCC TGCCCCACCC CGCGCACCTC GCCGACGAGG ATCACGTGGT
10200

CCCCGGCCAG GTGCCGGGCG TGTACCGCGC ACTCCAGCGT GACGAGCGCG TCCGCCGCGA
10260

CCAGCAGCCC CGAGCCCTCC AGCGCCACGA GCCCCCCGCC CCCGAACTTG TCCGCCCCGC
10320

GCGTCGCGAA CCGCGAGGCC AGCTCCCGCT GTCCGCTCCG CAGCACGCTC ACCGCGAACC
10380

GCGCGCACTC CTGGAACGCC CCGTAGGACT GCGCGCCCGT GCCCTGGCAC ACCGACACCA
10440

GCGGGGGAGC GAGCGACACC GAGCAGAAGG AACTCGCCGT GAAACCGCGG GGGGTGCCGT
10500

CCTCCTCGCG CGTCGTCACG AGGACCACCC CCGCGGGGAA GCACGACATC GCGGCGCGGA
10560

AGCCCTCGGC ATCGACCGGC CCGACCTGCC CCGTCCGCGC GGCCGTCCGT GTCTCGTTCA
10620

CGGGAGCTCC ACCACCTGGG CCGCGTAGTT GAGCCCCGCG CCGAACCCCA CGAGGAGCGC
10680

GCGGTCCCCG CTGCCCACCT CGCCGGAGTC GAGGAGCGCC TCCAGCGCGA GCGGGACCGA
10740

GGCCGCCGAG GTGTTCCCGG CGCGCACCAC GTCCCGCGCC ACGGCCGTGC GCTCCGGCAG
10800

GCCGAGCCGT TCGGCCATCA GCTCGATCAT GCGCAGGTTC GCCTGGTGCG GGACGAAGGC
10860

GTCCAGCGCC TCCGGCTCGA TCCCGCTCTC CCGCAGCAGT TCCGCCGCCC GCGGGGTCAC
10920

CTCGTCCATC GCCCAGCGGA ACACCCGCCG TCCGTCCATC CGCATCCACG GCCTGCCGAG
10980

CGAGGGATCG GCGGCGAACG TGTCCCAGCC CCTGTCCATC CGCAGCGCTC CGGCGTACCG
11040

GCCCGCCGCA CCACGCGCCG GCGGCGAGAT CCCCGGCCGC GCCGAAGGCC CCACCACAGC
11100

GGCTCCCGCC CCGTCCGCGA AGAGCACGGA GACGGTGCGG TCCGCACGCT CCACGAGATC
11160

CGTCATCCGC TCCGTGCCCA CCACCAGGAC GTGCCGGGCG CTCCCGGCGC GCACCGCGTC
11220

CGAAGCGAGC GCCAGCGCGT GGCAGAAACC GGCGCAGGCG CCGGACACGT CCAGCCCCGC
11280

CGCCGCCCGC GCGCCGAGGT CCTCGGCGAG GAGCACGGCG AGCGGGGGCG TCTGCTCCAG
11340

CCGCGACATG CTCGCCACCA GCACCAGATC GATCCCGTCC GGCAGCACCC CCGCCCGCGC
11400

GAGCGCCTTC TCGGCCGCCG CCCGCCCCAT GAACCGCAAG GTCTCGTCCG GCGCGGCGAA
11460

GCCGCGCGCA CGGATACCGC TGCGGGTCTC TATCCACTCC GGGGTCGACG CGACCGTGGC
11520

ACACAGTTCG GCGTTGTCCA CACTGCGCCG GGGCCGGTAA CCCCCCACCC CCAAGAGCCT
11580

GCTGTGCACG ACAGGCCGCG TACCGAGCAT CTCCGCCTCG CCGGTCACTG TCCCTCCCTC
11640

CCGCCAGGGG CCCGGTGCCT CCGGCCGCTG ACAGCCTCCG GGCCGCCTCT TGAGCCCGGA
11700

TCGAGCCGAG ATCGAGACCC CGGCCGGATG CTGGGGCCGT CACTCCCCGG CGCCGGCCGG
11760

GGGCCGAGGG GACCGGGCTC CGGGTCCCCA GGAAGGAACA CACGCGGCAT GAGCGCCTTC
11820

ACTCTCACCG AGTTCAAGAA GCTGGTCGAG CAGAGCTACG ACGCCGAATC GGCCGAAGCC
11880

CTCCACGGGC AGGCCCTCGA CACGAGCTTC ACCGATCTGG GTTACGACTC GCTGACGGTC
11940

TACGAGATCG TCACCCGCAT CCAGGACGAG CACGGAGTCA CCGTGCCCGA CGAGGAGCTG
12000

GACCTCCTCG ACACCCCGCG CGCCCTCATC GCGTACGTCG ACGCGCGCGC GGGTTCCCGC
12060

ACCTGACCCG ACGGGAGGGT GCGGGGAGGG CGGGGCCGGA GCGGGCGGGG CGCGGCGGCG
12120

GTACGACGCC GCCGCGCCCC GGGCGGCAGG CGCTCAGCCC CCGTCCCGTA CGACCTGACC
12180

CGCCTGTTGG AGCGCGGCGC GAAAGGCCCG CACGTCGTCC GCGGGGTCGC CGGGACGCGC
12240

GGGCAGGAGC CGAACGACAC GGGCCGCGCC CGAGCGGACG GCCGGATGAC GGCGCGCCGG
12300

GGCGCCGAGA CTGCCGTCCG GCGAGGCGTC GAGCAGCACC ACGCCACGCC CGCCGCCCGT
12360

GGTCGTCGCC GTCGCCGTCG CCGTCGCGAG CAGGTTCCCC AGCGCCGGCC AGTACAGGAC
12420

GGGGGCGGCG AGCTGACCGC ACCAGAACGC GGCGCGCACC GCCTCGCCCG GCGTGAGCGC
12480

GCGACCCGTC CGGCCCGACC ACAGGGGCAC GGCGGGTGGC CGCCACCGCG CGACCCGTTC
12540

CGCGCTCGCC CTGGTCAGAC GCCGCGCCGC CCCGGCGAGG GCGGGGGAGT GGAAGGGTAC
12600

GTCCGCCTGG AGGCGGCGCG CCGTCAGCCC GTCGTCGGCC AGGCGCCGCG CCACCGCGCG
12660

CAGCGGCGCC TCCGGCCCGC ACAGCACCGT CTGACGCGGC CCGTTCACCG CGCCCACCAC
12720

CACGTCGGCC CCCACGTACG GGCGCAGGTC GTCCACCGGC GCGGCCACGG CGAGCATGCC
12780

CCCGGACGGC GCCGCGCGCA GCACCGCGTC CCGTTCGGCG AGCAGCCGGA GCGCCGCCCC
12840

CGGCGCGCAC ACCCCGGCGA GCGCGGCGGC GGCGAGTTCG CCGACGCTGT GCCCGAGCAA
12900

CAGGTGCGGC GCACCCTGCG CCCCCGCGCC GAGCGCCCGC CCGAGCCCGT ACCCCACGGC
12960

GAAGAGCAAC GGCTGCGCGA CAGCGGCCCG TTCCAGCGCC TCCTCGCCGC CCGCCCCGTC
13020

CAGCCACCGC GCGCGCAACT CCTCCCCCCA GGTGCCCCAC ACGGCGAAGC ACTCCTCCAC
13080

CGCGGTCGTG AACTCCGGCA CCACGCCGTG GAGTCCGGCC GCCATCCGGG CCCGCTGCGC
13140

GCCCTGACCG GGCAGCAGCA GGGCCACCAC CGGGGCGCCC TCCTCCTCCG TACCGCCCTC
13200

GCTCCGCACT CTTCCGCCTC CTCGTCCCGC CGACTCCTGC CGGTACCGAG CCTCGCCCTT
13260

GCCGCTCGAC GGCCGGTGGA CTCCCGCTCC AGCGCGGCCC GCCCGGGGCG CCACCGAGCC
13320

TCAAGCCCGC TTCGAGGGAC GTCTTCCAAG GTCGGGTCGA GGCCGTGAAC GGCACCGCGC
13380

CGTCCAGGAC GCCCAAGGGG GTCGTGCAGT GAACCGACAA GTCGCCGTCA CCGGCATCGG
13440

CGTGGTGGCT CCGGGAGGGA TCGGGCGCAA GCCGTACTGG GAGCAGCTCA CCTCCGGACG
13500

CACCGCCACG CGCGCCATCT CCTTCTTCGA CGCCTCGCCC TTCCGCTCGC GGATCGCCGC
13560

CGAGGTCGAC TTCGACCCCG CGGCGGCGGG CCTGAGCCCG CGCGAGGTCC GCCGCATGGA
13620

CCGGGCCGCG CAGTTCGCCG TCGTGAGCGC CAGGGAGAGC CTCGCCGACA GCGGACTCGA
13680

CGTCGCCGAC CTCGACCCCC ACCGGATCGG GGTGAGCATC GGCAGCGCCG TGGGCGGGAC
13740

CACCTCGCTG GAACGCGAGT ACCTCGCCCT CAGCGACAGC GGGCGACAGT GGGAACTCGA
13800

CCTCTCCTAC CTCTCGCCGC ACCTCTACGA CGCCTTCACC CCCAGCTCGC TCGCCCGCGA
13860

GGTGGCCGGG GTGATCGGCG CGGAGGGGCC CGCGGCAGTC GTCTCCACCG GCTGCACCTC
13920

CGGCATCGAC TCGCTCGGCC ACGCCCGCGA CCTCATCGCC GAGGGCAGCG CCGACGTCGT
13980

GCTCGCGGGC GGCACCGACA CCCCCATCTC GCCGATCGCC GTCGCCTGCT TCGACGCCAT
14040

CAAGGCCACC TCGCCCAGCA ACGACGACCC GGCGCACGCC TCGCGCCCCT TCGACAGGGA
14100

ACGCAACGGC TTCGTCCTCG CGGAGGGCGC CGCCGTCCTC GTGCTGGAGG AGCTGGGGCA
14160

CGCGCGGGCC CGCGACGCCC ACGTCTACGC TCTCGTGTCC GGCTACGCCA CGCGCTGCAA
14220

CGCGTACCAC ATGACCGGGC TCACCCCGCA CGGCAGGGAG ATGGCCGAGG CGATCCGGCA
14280

CGCGCTCGCC GAGAGCGGGA CCGACCCCGC CGCCGTGGAC TACGTCAACG CCCACGGCTC
14340

CGGCACCAAG CAGAACGACC GGCACGAGAC CGCCGCCTTC AAGGCCACGC TCGGCGAGCG
14400

GGCCAGGAGC GTGCCGGTCA GCTCCATCAA GTCGATGATC GGCCACTCGC TCGGTGCCAT
14460

CGGTTCGCTG GAGATCGCGG CCTGGGCGCT CGCCATGGAG TACGGCGTGG TGCCGCCCAC
14520

CGCCAACCTC GACACGCCCG ACCCCGAGTG CGACCTCGAC TACGTGCCCC ACGAGGCGCG
14580

CGAGACGCGG GTGGACAGGG TCCTGAGCGT CGGCAGCGGC TTCGGCGGTT TCCAGAGCGC
14640

CATGGTCCTC ACCCGGGACA CGGGCGCCCG CCTCCCGACG GCCTGACACC CGCTCACCGC
14700

ACGCCCCGCA CCGGCCCGGA CGGCCGGCCA GGTCCCGCAC CGCTCGCACA CGGAAGGCAC
14760

CCGCCCATGA CCACCGCCCC CTCCCGCACC GCCCAGGGCG CCCCTCCCGG GGCCGCCCTG
14820

CCCCCCGTCT TCACCGGCAT CGGCGTCGCC GCCCCCAACG GGCTCGGCAC CGAGGAGTGG
14880

TGGGCGGCCA CCCTGCGCGG GGAGCACGGT CTGCGGCCCG TCACGGAGTA CGACGCGAGC
14940

GGCCACCCCG GTGGACTCGT CGGCCGCGTC CCGGACTTCG ACGCCGCCCG CCACCTGCCC
15000

GGCCGCCTCC TGCCGCAGAC CGACCGGGTC ACCCGGCTCG CGCTCGTCGC CGCCGACGAG
15060

GCACTGAAGG ACGCCGCCGT CGACCCGGCC CGGCTGCCCG AGTACGGGGC GAGCGCCGTC
15120

ACCTCCAACG CCACCGGCGG CTTCGAGTTC ACCCACCGCG AGATCCGCAA ACTCTGGACC
15180

GAGGGCCCGG CCCGCGTCAG CGTCTACGAG TCCTTCGCCT GGTTCTACGC CGTCAACACC
15240

GGCCAGATCT CCATCCGGCA CGGCATGCGC GGCCCCGGCG CCGTCGTCGT GGCCGACCAG
15300

GCGGGCGGCC TCGACGCCCT CGGCCAGGCC CGCCGCGTAC TGCGCAAGGG CGGGGTGCTG
15360

GCGGTGAGCG GCGGCGTGGA GTCCGCGCTC GACCCCTGGG GCCTGGCCGC CCACGCCTCG
15420

TCGGGCACCC TCAGCCGCTC CGGCGACCCG GCCACCGCCT ACCTCCCCTT CGACCGGCGT
15480

GCCCTCGGCA CCGTCGTCGG CGAGGGCGGC GCCCTCCTCA CCCTGGAGAC ACCCCGGCAC
15540

GCCGAGGAGC GCGACGCACC CCGGATCTAC GGCGAACTCG CCGGGTACGC CGCCACGTTC
15600

GACCCGCCCG CGGGCTCCGG ACGGCCCCCC GGCCTCGAAC GCGCCGCGCG CCTCGCCCTC
15660

GCCGACGCGG GCCTGGCACC CGGGGACGTC GACGTCGTCT TCGCGGACGC GGCGGGGCTC
15720

CCCGCCGCCG ACGCCGCCGA GGCCGCCGCC CTGCGCGCGC TCTTCGGCCC CGGCGGCGTT
15780

CCGGTGAGCG TGCCGAAGAC CCAGACCGGG CGGCTCGCCT CGGGCGGCCC GGCCCTCGAC
15840

GTCGCCGCCG CCCTCCTCGC CCTGCGCGAC GGCCTCGTGC CCCCGGCCGT CCACCTCGAC
15900

GAGGTCGATC CCGCGTACGG CCTCGACCTC GTCCGCGACA CCCCGCGCGC CCTCCCGCTG
15960

CGCACGGCGC TCGTCCTCGC GCGCGGCCAC GGCGGCTTCA ACGCGGCCGT CGTCGTCCGC
16020

GGGCGGCGGC GGCCCCGTAC CGCCTGAGGG CCTGCCCCGG GTGCGCCGCC GCGGCCGCAC
16080

CCGCCGTCCA CCGCCCCGCA CACCCCACAA GGAGTTCCCA TGAGCGCACT GACCGTCGAC
16140

GACCTCAAGA AACTGCTCGC CGAGACCGCC GGGGAGGACG ACAGCGTCGA CCTCGCCGGA
16200

GAACTCGACA CGCCCTTCGT GGACCTCGGC TACGACTCCC TCGCGCTGCT GGAGACGGCC
16260

GCCGTGCTCC AGCAGCGGTA CGGCATCGCG CTGACTGACG AGACGGTCGG CCGGCTGGGC
16320

ACCCCGCGCG AACTGCTCGA CGAGGTCAAC ACCACCCCGG CCACCGCCTG AGCGGGCGGC
16380

GCACGCGAAC GAAGGGTCCG GCCCCACCCC CCGCGCGGGG GGTGGGGCCG GACCCTTCGT
16440

TCGCGGCGGA CCTCACAGCA AGGTGCGGTC CTGGGTCACC TGCCGCGACA GCGTGCGCAG
16500

TTCCCCGCCC TCCCGCACCA GGACGTCCCG TACGACGCAA CTGGGACCGA TCACCGGGAG
16560

CGCCGCGTCC GGCCGGGTCA CCACGACCAG CGCGTAGAAC TCCGTCTCGA CGGCGCCGTC
16620

CTCACCCGGG CGTACGTCCA GCATCGAGAA CCAGTGCCGC CGCTGGACGG GGTCGTCGGC
16680

GAACCGGCGG GCGTTGAAGC CGCGCACCTC CTCCGCGATC GCCGCCCTGC CCCGGGCCGC
16740

CGGGGTGTCG GGGGAGTGGG CGAAGACGCC CTCCGCCGCG AAGGTGGCCG CGAACTCCTC
16800

GGCCCGTACC GCGTCCAGGT GGTGTGCCTG GCGCGCGTAG AACGCCTGTA CCTCGGTGTA
16860

GAGCGCCGCG GAACGCGCCG CGTCGCCCGG CAGGGACCCG GCCCTCACGT CGTCCGGCGG
16920

GGTGGTGGTC GTCACGGGAG CCTCCCAGAA GGTGTCTGAC CTGTCCCGGC AAGCCTCCGC
16980

GCCGTGGCTC GACGGCCCCT TGAGCCCCCC TCAAGGGAGC GCCGCGAGGC TGGCACCAGC
17040

ACCGCGGCGT CACCACCGAC GCCCCCGCGC CGAGGAGGAA GGCCATCATG ACCACAGCAG
17100

CTCCGCACAC CCGCCCCGGG GAGGCAGGCA CCACCCGGGG ACCCGCTCTC GTGACCGGCG
17160

CGACCCGGGG CATCGGCCTC GCCGTCGCCG AGGCGCTCGT CGCGCGCGGC TATCCCGTGG
17220

TGGTCTGCGC CCGCGACGCC GAGGCGGTCG CGCGCACCGT CAAGGAGCTG GCAGCGGGCG
17280

GCGCCCGCGT CGAGGGCGTC GTCGCCGACG TCACCGACGC CGCCTCCGTG CACGAACTCG
17340

TCGCCACCAC CGTCGCCCGC TTCGGCCCCG TCGAGGTCCT CGTCAACAAC GCGGGCCGGT
17400

CCGGCGGCGG AGTGACCGCC GAACTCAGCG AGTCCCTGTG GGACGACGTC ATCGCCACCA
17460

ACCTCAAGAG CGTCTTCCTG GTCACCCGGG AGGTGCTCAC CACGGGCGGG ATGACCGGGC
17520

GCGGCCGCGG CGTCGTCAAC ATCGCCTCCA CGGGCGGCAA GCAGGGCGTC GTCTTCGGCG
17580

CCCCCTACTC GGCGTCCAAG CACGGCGTCG TCGGCTTCAC CAAAGCCCTC GGCCTCGAAC
17640

TGGCCCGGAG CGGCATCACC GTCAACGCCG TCTGCCCCGG CTACGTCGAG ACGCCGATGG
17700

CCGCCGGAGT GCGCCGCCAC TACGCCGACC TGTGGGACGT CACCGAGGAG GACGTGCTGG
17760

CCCGCTTCGA GGCGAAGATC CCGCTCGGCC GGTACACCCG CCCCGACGAG GTCGCCGCCC
17820

TCGTCGACTA CCTGGTCACC GACGCCGCCG CGGCCGTCAC CGCCCAGGCC CTCAACGTGT
17880

GCGGCGGACT GGGGAACTAC TGATGACCAC CACCACGACC GGGCACCAGC GCCCCGGCTC
17940

CGCCGAACAC TCCGCGCGCC TCGCCGCCCC GCCCGCCTCC GCCTACGAAC TCGTCGCCGA
18000

CGTGACGCGC TGGCCCCTCC TCTTCACCCC GTGCCTGCAC GCCGAGGTGC TGGAGAGCGG
18060

TCCCGGCACC GAACGCGTGC GGCTGTGGGC CCTCACCGGC GAGCAGGTGC GCGGCTGGAC
18120

CTCGCGGCGC ACCCTCGACA GCGAGGGCCT GCGCGTCGGC TTCCGCCAGG AGGACAGCGC
18180

CCCGCCGCTC GCCGCGATGG GCGGGGAGTG GCGCTTCACC GAGGAGGGCG AGGACACGCG
18240

CGCCGTACTC GCGCACGACT GGACGCTCAC CGAGCCGGGC GCCGCACCGC ACCGCTGGGT
18300

CACCGAGACA CTCGACCGCA ACAGCACCGC CGAGATCGGG GCCGTGACCG CCTGGGCCGC
18360

GCGCACCCAC GCGGCGGGCG GCGCCGACGC GCTGCTCTTC TCCTTCACCG ACAGCCTCGA
18420

CATCGCCGCC CCCGCGCCCG ACGTGTACGC CTTCCTCGAC GCCGCCGACC AGTGGCCCGC
18480

GCGGCTCCCG CACGTCAGCC GGGTCGCCTT CTCGACCACC CCGGCGACGC CGCTCACGGC
18540

GGGCGCGGAG GTGCAGCACC TGGAGATGGA GACGCGCGCG GACGACGGCA CGCGCCACCT
18600

CACCCGCTCG ATCCGCCTCG GCTTCGCGGG CCGTCTCCTC GTCTACAAGC AGACCACGTT
18660

GCCCGCCCCG CTCCTCGGCC ACGCCGGCTC CTGGGCGCTG GAGCCGCTGC CCGGCGGCGG
18720

CACCCGGGTC ACCGCCCGCC ACCGGGTGGC ACTCGACCCG GACGCCGTGA CCGAACGCTT
18780

CGGCGCGGGG ACCACGCTCG CGGCGGCGCG GGACACGGTA CGGGCGCTGC TCGGCGGCAA
18840

CAGCAGGCGC ACCCTGGAGG CGGCCCGCGC CCACACCGAG TCGGCGGGGG AGCGATGACC
18900

ACGACCGGCC CCGCGACTCC CCTGGCCCCC GGGCCCGCAT CCGCCGAGAC CGTCGCGCTC
18960

GCCGACGGCG TGCACTCCTG GCTCCAGCCC GACGGCGGCT GGTGCGTCAG CAACGCGGGC
19020

ATCCTGCTCG CGCCCGACCG CGTCGCGCTC GTCGACACCG CCGCCACCGA GGCGCGCTCC
19080

CGCGCGCTCG GCGCCGCGGT CGCCGGACTC AGCCCGCATC CCGTGCGACT GCTCGTCAAC
19140

ACCCACTTCC ACGGGGACCA CAGCTTCGGC AACGGCATCC TCGGCAAGGA CGCCGTCATC
19200

GTCGCCCACG AGCGCACCCG CACCGAGATG GCCGAGGCCG GACTCGGGCT CACCGGACTG
19260

TGGCCGGGCG TGGACTGGGG GCACGTGGAC CCGGTCCTGC CCCAGCTCAC GTACCGCAGG
19320

CGCCTCACGC TCCACCACGG GGACCTCCGC GTCGAGCTGA TCCACCCCGG CCCGGCGCAC
19380

ACCACCAACG ACACCCTGGT GTGGCTCCCC GAGCAGCGCG TCCTGTTCGC GGGGGACGTA
19440

CTCCTGCCGG GGGCGACGCC CTTCGTGCTG ATGGGCTCGG TGACCGGCTC GCTGCACACC
19500

CTGCGTCTGC TGCGCCGCCT CGGCCCCCGC GTCGTCGTCG GCGGGCACGG CCCCCTCGCG
19560

GGGCCCGAGG TGATCGAGGA GACGGAGCGC TACCTGCTGC GGCTGCGGCG CATCGCCACC
19620

GAGGGCCACG CCGCGGGGCT CACCCCGCTG GAGGCGGCAC GGCGCCACGG CCCCGGGCCC
19680

TTCGCCCACT GGAGCGAGCC GGAACGACTC GCCGCCAATC TGCACCGGGC GTACGCCGAA
19740

CTCGGCCCCG CACCACTGGG CACGCCGCTC GACGTCCTCG CCTGCTTCGG CGACCTCATC
19800

GCCTACAACG ACGGCGAGCT GCCGGTCAGC CACGCCTGAA CAGCCCGGCC ACCGGCCGCT
19860

CACGGACCGC GCCGCGCCCT GCCCCGGTCC GCCCCGCCCC TGCCCGCGCG AACGACCGGC
19920

GCGGGCCGCC CCCGAGGGGA ACGGGCCGCG CCGGTCATCC GTGCGGACAG GGCGAGCAGG
19980

CCGCGCCGCT CACCCGCCGA CCACGGTGGG CGGTCCGGGC GCGAAGCTCG CCGCGTGGTC
20040

GCGCACCCAA GTGGCGAAGG ACCGCGCGGG CCGACCGGTC ACGTCGGTGA CACCCGGGCT
20100

CACGGCCGGT GGGTGCTCCG TGAAGAACCG CTCGACGCCC GCGAGCGCCC GCGCGTACGG
20160

CTCGGGGATG TGCCGGGCCG TCAGCTGCAC CCACTCCTCC TCCGGCACCG GCTCCACGCG
20220

CAGCGGGCGG CCCAGTTCCG CGGCGAGCAC GGCGGTCCGC CCGGGCAGCG TCAGCACCTC
20280

GGGACCCGAG AGCGGCACCG CCTTTCCGGC CCACCGCTCC ACGGGCGCCG TGAGAACCGC
20340

GGCGGCCACG TCGGCGATGT CCCGCGGGTC GATCAGCGGC ACCGCGAGCC CGGCGTGCGG
20400

GACCCGCACC ACACCGTCCC CGTGGAGCGC GGGCGCCCAC AGCAGCGCGA GCGAGGCGAA
20460

GGCGCCCGGA CGCAGGAACA CCCAGGGCAC CCCGCTCCCG CGCACGTGCC GCTCGCCGCG
20520

GACGTGCTTC TGGAACATCG GGTTGTCGTA CTCCGGGTGA GCCGCCGCCG TCGCGGAGAG
20580

CACCACCACC TCGCGCAGCC CCCGCGCCTT CGCCGCCGCC CGCGCGAAGG GCGCGCCGGC
20640

CTCGTCCTGG AGCAGCAGGA ACGCCCGCTC CACGCCGTGC AGCGCCGCCG CGAGCGAAGC
20700

CGTGTCCGCC AGGTCCCCGA CGACCGTCTC GGCGCCGACC GCGGCGAGCG CCCGCCCGCG
20760

CGCGGCCGAG CGCACCAGGG CGCGCACCGG CACCCCGGAG TCGAGCAGCC GGGACACGAG
20820

GCGCCCCCCG ACCGCGCCGC TCGCCCCGGC CACCAGCACT GTTCCCGGCA GTGGTCCCGC
20880

CATCGTTCCC GTCACCCTTC CCGTCACCGG TTCGCTTCCT GTACCGCCCA GGCGCCGTAG
20940

GCGGCGCCCG CGGCCTCGGG GGAGAGCGCG ACGTGCGAGG CGCCGGTCTG CACGTCCCGC
21000

CAGAACCGCT GCACGGGGTC GTGCGGGCTC TGCCCGGACG TCCCGCTCGC ACGGACGAGC
21060

CGCTGCACCG CCCCGACGGC CAACTCGGCG GCGAGGGCCG TGTCCCGCTT GCCGCGCACC
21120

GCGGCGGCGG GCTCGGGAAG GGAAACGGTG CCGTCGGCCG CCGCCGCCGC GCGCAGCACG
21180

AGCAGCTCGG CCGCGTCGGC CTCCCCGGCC GCCCGGCTCA GCTCCCCGAG TGCTCCGGGC
21240

GAAGGCGCCC GCCCCTGCGC GGCGCGCTCG GTCCACGCCC GCAGGGCGCC CCGCGCCGCG
21300

CCGACGAGCG GCGTCACGAC GGGCAGTGCC CCGACCCGCG CCATCCGCAC CGTGGGCACC
21360

GCGCCCGCGA CGCGGGGGCG CTGCCCCGCG AGGACACGGG TGCGCGGGAA GGAACGGTGC
21420

GCGGGGACGA GGACGTCGTC GAGCACGAGG GTGTCGCTGC CCGTCGCGCG CATCCCCGTC
21480

GTGTTCCAGG TCCGTTCGAC GCGGGGTGCC GCGCGCGGCC CCGCGAAGAA CCGGACCTCG
21540

GGGCCCTCCT CGCCCTCGCC GCCCGGCACG GTCGCGCAGG CGAGCACCCA GGCGGCGTGC
21600

CGCACGCCGC TGATGTAGGG CCAGGCCCCG TCGAGCCGCC AGCCGCCGGG GACGGGCGTC
21660

ACGGTCCCCG AGGGGACGAG GGCGCCCGCG AGCAGCGCGT CCGGGCCCTC GGCCCACACC
21720

TCGGCCTGCC CCTCCTCGGG CAGGTACGCG GCGTACCGCC CGGCGTAGGC GGAGAGCGAG
21780

GCCGCCCACG CGGCCGAGGT GCAGCCCGTC CCGAGGCGCA GGACGGCCTC GGCGAGTTCC
21840

CCGAAGCCGC CTTCGGCGCC GCCGTGGGCC GCGGGCACGA AGTGGCGGGC GAAGCCCGCC
21900

GCCACGATGC CCGCGGACGA CTTCCCCGGC GAGACCCCGC TCCTCCTCAC CGCGGCGCGC
21960

GTGCCCGGCC GCGAGCGCGG CGAGCGCCCC GGCCCGTTCC GCGAGCGGTC CGGGGGGCGT
22020

CCCGGCCCCT CGTCCGGCGC CCCGGTCCGC TCGGCGTGTG CCGGTGCGGC GAGGGTGGTC
22080

GATTGCTCCG TCATCGGTAC GTCACCTCCA CGCCGAACAT CTCCCGGCCT GCTGGAGGCG
22140

CGCTCGACTC GCGCTGGACG AGGCGGGGCG GCGAGCCCTC AGGCGAGGGA GTCCCCGGCC
22200

GGTGCGCCGG GCGTGGGCAC CGGGGCGTAG ACCTGCGGCG CGGTGTGGGC CTCGGCGCGC
22260

AGCCGCGCGA AGGCGGGCCG GATGCCGGGG GCGCTCGCCG CCGCCCGGTG GGCGGCGGCG
22320

TCGCTCCAGT CGGCGAGCAT GGCCCACCGC TCGGGGTGGT CGGGCGAGCG GTGCAGGCGC
22380

AGGGAGAGGA AGCCGGGCTG CGCGCGCATG TGGGCGCAGA TCTCTTCGAG GACCGCGAGG
22440

AAACGGCTCT CGTCGCCGTG CAGGAGCATC TGGTTGATCA TGGTCACCAT GTGTCCACCC
22500

GACACCGTCC CGCTTGAGTC CCGCTTGAAG AGGCACCGCC GACACGCGGG CGGGTGCGGG
22560

GAGTGAGGGG TACGGAGGCG TCCGGTGACG CGTACGGGGC GTCCGGTGAC GCTTTCGGAG
22620

GCGGCCGGTG ACGCGCGGGG GCCGCCGGTG GGGGTTTCTC ACCGGCGGCC CCGGGCCGTG
22680

CGTGCTCAGC CGAGCGGTGC GGGGAAGGTC GGGTACTCGA CGCCCGAGAC GTGCTGGACG
22740

ACCCGTACGA CCTGGCACGA GTAGCCGAAC TCGTTGTCGT ACCAGAGGTA GAGGATCGCG
22800

TTGTCGCCGT CGACCTTGGT CGCCCCGGCG TCCACGATCG AGGCGTGGCG CGAGCCGACG
22860

AAGTCGCTGG AGACGGCGTC GGCGGAGGTG GTGAAGTCGA TCTGGCGGCG CAGCGGCGAG
22920

GTGAGGGAGA CCTCGCGCAG GTGGGTGAGG ACCTCCTCGC GGCTCGTCCC GCGGCTCAGC
22980

CGGAGGTTCA GGATCGCGAT CGACACATCG GGGACGGGAA CGCGGATCGA GCTGCCGCTG
23040

ATCTTCGCCC CGAGGTCCGG CAGAGCCTTC GCGACGGCCG AGGCGGCACC CGTCTCGGTG
23100

AGGACCATGT TGAGCGGCGC GGACCGCCCT CGCCGGTCGG ACTTGTGGTA ATTGTCCAGC
23160

AGGTTCTGGT CGTTGGTGAA GGAGTGCACC GTCTCGACGT GGCCGCTCTC GACGCCGAAC
23220

TCGTCGGCCA TCGCCTTGAG CGGCGGCACG ATGGCGTTCG TCGTGCAGGA GGCGCAGGAG
23280

ATGATCCGCT CGTCCGGCTT GATCGTCTCG TGGTTCACGC CGTGCACGAT GTTCGGCACG
23340

TCGCCCTTGC CGGGCGCGGT GAGGACGACC TTCGCGACAC CCGGGCGCAG ATGCTGCGAG
23400

AGGCCCGCGC GGTCGCGCCA GCGGCCCGTG TTGTCGACGA GGATCGCGTC GTTGATGCCG
23460

TAGGCGGTGT AGTCCACGGT CGCCGGGTCG TCGGAGTAGA TGAACCGGAT CGCGTTGCCG
23520

TTCGCGATGA GGGTGTCGTT CTCCTCGTCC ACGATGATCG TGCCCTGGAA CTGGCCGTGC
23580

ACGGAGTCGC GGCGCAGCAG TGAGGCGCGC TTGACGAGGT CCTGACCGGC GGTCTTGCGG
23640

ACCACGACGG CGCGCAGACG CAGGCCGTTG CCCGAGCCGG CCTTCTCGAT GAGCAGCCGG
23700

GCGAGGAGCC GCCCGATCCG CCCGAAGCCG TAGAGGACGA CGTCGCGCGG GGCGGCGCGC
23760

TCGATCTTCC GCTCGCCGGT CGCGCCCTCG ACGGGCCGCG CGGTGAACTC GGCGACGCCC
23820

AGGCCCCGGT CGTCGCTCTT GTAGGTCTCG GCGAGCATGC CGATGTCGAT CTGGGAGGGG
23880

CCGAGGTCGA GCGTCGTGAG CGCCTGGAGG AACGGCATGG TCTCGGTGAC CGACAGCTCC
23940

TCGCCCGCGA TCTGCCGGGC GAATCGGTGG GTCTTGAGAA TCCCGACCAC CGACTTGTTC
24000

ACCAGCGAGC GGCTGTGCAG CAGCACGGTG ACGTCCCGTT GCCGCTGGAG CCTGCCGATG
24060

ACGGGGATCA TCGATTCCGC GATCTCCTCG CGGGTCTTCC AGTTGGTGAA CGAGTCCTCG
24120

TTGACAGTCA CAGATCCATC TTTCGAGCTA GGCGGCGCTC ATATGTTAAC CCGATGGGTG
24180

TGATCATCTG CCGGGCGCGG GTGTGATGTA CGACCAGCTC GGGGCGGGTG CGGGACGTCG
24240

GGGATGGGGG CGCGGAGCAC CGGGTGCGGG GTGTGGGTGG CCGGTGGGGG GACGTGCGGC
24300

GGGGGTGCAG TACGTGCGGT GCGGGTGCGG AACGTGCGGC GAGGTGCGGA ACGTGCGGTG
24360

CGGGTGCGGA ACGTGCGGC
24379

## Description

The present invention relates to the identification, isolation, and sequencing of the genes associated with the microbial production of the antibiotic frenolicin B, particularly from *Streptomyces roseofulvus*.

Polyketides are a large, structurally diverse family of natural products, displaying a broad range of biological activities including antibiotic and pharmacological properties. For example, polyketides are represented by the antibiotics tetracycline and erythromycin, the anticancer agent daunomycin, the immunosuppressants FK506 and rapamycin, and veterinary products such as frenolicin, monensin and avermectin.

Polyketide synthases (PKSs) are multifunctional enzymes related to fatty acid synthases (FASs). PKSs catalyze the biosynthesis of polyketides through repeated Claisen condensations between acylthioesters, usually acetyl, propionyl, malonyl or methylmalonyl. Following each condensation, they introduce structural variability into the product by catalyzing all, part, or none of a reductive cycle comprising a ketoreduction, dehydration, and enoylreduction on the β-keto group of the growing polyketide chain. After the carbon chain has grown to a length characteristic of each specific product, it is released from the synthase by thiolysis or acyltransfer. Thus, PKSs consist of families of enzymes which work together to produce a given polyketide. The controlled variation in chain length, choice of chain-building units, and the reductive cycle, genetically programmed into each PKS, contributes to the variation seen among naturally occurring polyketides.

*Streptomyces* is an actinomycete producer of aromatic polyketides; in *Streptomyces*, it is known that PKS-mediated synthesis relies upon the products of at least three PKS open reading frames (ORFs). ORF1 encodes a ketosynthase (KS) and an acyltransferase (AT) active site; ORF2 encodes a protein similar to the ORF1 product but lacking the KS and AT motifs; and ORF3 encodes a discrete acyl carrier protein (ACP).

Frenolicin B is a naphthoquinone polyketide which is widely used as an anticoccidial in domestic animals and birds. Iwai et al. in *J. Antibiotics*, **31**:10, 959-965 (1978) reported the isolation of two antibiotics of the frenolicin group, AM-3867 I and II, from the fermentation broth of *Streptomyces roseofulvus* strain No. AM-3867, a soil isolate. AM-3867 I was found to be a new antibiotic subsequently denoted frenolicin B, while the latter was identified as deoxyfrenolicin.

Bibb et al. in *Gene*, **142**, 31-39 (1994) reported the cloning of a 10.2kb fragment of DNA from *Streptomyces roseofulvus*, which contains polyketide synthase(PKS)-encoding genes (*fren*) presumed to determine production of the frenolicins and the nanaomycins. A 5530bp continuous segment of this DNA was sequenced. Analysis of the sequence revealed five complete open reading frames (ORFs) transcribed in one direction (ORFs 1,2,3,5,4) and one (ORFX), located between ORF3 and ORF5, transcribed in the opposite direction. The deduced amino acid sequences of ORFs 1,2,3,4 and 5 closely resemble the sequences of known components of type-II PKSs from other *Streptomyces* species: putative heterodimeric (ORF1+2) ketosynthase, acyl carrier protein, cyclase and ketoreductase, respectively. A resemblance between the N-terminal and C-terminal halves of the ORF4 product, also discovered in the corresponding genes from other isochromanequinone antibiotic producers, suggests a possible origin of the cyclase-encoding gene by duplication. ORFX appears to represent a novel class of genes of unknown function present not only in the *fren* cluster, but also in other clusters of aromatic antibiotic biosynthetic genes in *Streptomyces* species. Attempts to introduce these genes into strain AK24158 to prove, by gene disruption, that the *fren* genes are indeed those that encode the frenolicin PKS were reported to be unsuccessful.

It is an object of this invention to provide the full sequence of the gene cluster encoding the frenolicins and to isolate and identify the open reading frames of the cluster.

It is also an object of this invention to provide engineered host cells transformed with any or all of the isolated frenolicin PKS genes.

It is furthermore an object of this invention to improve yields in the biosynthetic production of frenolicins.

This invention provides an isolated gene cluster comprising SEQ ID NO:22 encoding proteins which catalyze the biosynthesis of frenolicins in prokaryotio cells, and modified gene clusters having sufficient functional biochemical equivalence to SEQ ID NO:22 to likewise encode proteins which catalyze the biosynthesis of frenolicins. Also included are vectors comprising the gene cluster operatively linked to expression control sequences and host cells transformed with such vectors.

Also provided are five isolated gene subclusters responsible for particular aspects of the frenolicin synthesis:
an isolated first gene subcluster encoding an efflux pump useful for the production of frenolicins, comprising genes encoding proteins as set forth in SEQ ID NOS: 1, 2, 3, 4, and 6, and biochemically equivalent muteins of said proteins;
an isolated second gene subcluster encoding butyrate starter synthases useful for the production of frenolicins, comprising genes encoding proteins as set forth in SEQ ID NOS: 8, 9, 10, and 11, and biochemically equivalent muteins of said proteins;
an isolated third gene subcluster encoding polyketide synthases useful for the production of frenolicins, comprising genes encoding proteins as set forth in SEQ ID NOS: 12, 13, and 14, and biochemically equivalent muteins of said proteins;
an isolated fourth gene subcluster encoding a hemiketalase, a ketoreductase, and cyclases/dehydrases useful for the production of frenolicins, comprising genes encoding proteins as set forth in SEQ ID NOS: 15, 16, 17, and 18, and biochemically equivalent muteins of said proteins; and
an isolated fifth gene subcluster encoding a keto/enoyl reductase and an hydroxylase, useful for the production of frenolicins, comprising genes encoding proteins as set forth in SEQ ID NOS: 19 and 20, and biochemically equivalent muteins of said proteins.

In another embodiment, each gene may be independently inserted into vectors operatively linked to expression control sequences.

In another embodiment a method for the biosynthesis of frenolicins by a) transforming a host cell with a recombinant vector encoding the gene cluster of SEQ ID NO:22; b) expressing said vector in said host cell; and c) isolating the frenolicins produced therefrom is provided.

Also provided is a method for the recombinant biosynthesis of the antibiotic frenolicin B by a) transforming a host cell with a vector encoding for the gene cluster of SEQ ID NO:22; b) expressing said vector in said host cell; c) isolating the frenolicins produced therefrom; and d) oxidizing the mixture of frenolicins to convert substantially all of the isolated product to frenolicin B.

Also provided are isolated proteins of SEQ ID NOS:1 through 20 and biochemically equivalent variations thereof and isolated genes encoding such proteins.

It is furthermore an object of the present invention to provide a DNA sequence comprising at least one of the following DNA sequences:
a) base 636 to 2948 of SEQ ID NO:22.
b) base 2945 to 3916 of SEQ ID NO:22.
c) base 4020 to 4844 of SEQ ID NO:22.
d) base 4841 to 6415 of SEQ ID NO:22.
e) base 6533 to 7183 of SEQ ID NO:22.
f) base 7344 to 8897 of SEQ ID NO:22.
g) base 9164 to 10012 of SEQ ID NO:22.
h) base 10621 to 10105 of SEQ ID NO:22.
i) base 11628 to 10618 of SEQ ID NO:22.
k) base 11809 to 12066 of SEQ ID NO:22.
l) base 13209 to 12154 of SEQ ID NO:22.
m) base 13409 to 14686 of SEQ ID NO:22.
n) base 14767 to 16047 of SEQ ID NO:22.
o) base 16120 to 16371 of SEQ ID NO:22.
p) base 16935 to 16453 of SEQ ID NO:22.
q) base 17088 to 17903 of SEQ ID NO:22.
r) base 17903 to 18898 of SEQ ID NO:22.
s) base 18895 to 19839 of SEQ ID NO:22.
t) base 20907 to 19990 of SEQ ID NO:22.
w) base 22094 to 20904 of SEQ ID NO:22.
or a DNA sequence which codes for a biochemically equivalent variation of a protein encoded by a DNA sequence as specified under a) to w) and more specifically such a DNA sequence which comprises all DNA sequences as specified under a) to w), preferably organized in the same way as in SEQ ID NO:22, or such DNA sequences wherein at least one of the DNA sequences specified under a) to w) is replaced by a DNA sequence which encodes a protein which is a biochemically equivalent variation thereof and even more specifically a DNA sequence which comprises the DNA sequence of SEQ ID NO:22 or a DNA sequence which encodes proteins which are biochemically equivalent variations.

It is furthermore an object of the present invention to provide a vector comprising such DNA sequences operatively linked to expression control sequences, a host cell transformed with such a vector, specifically wherein the host cell is a member of the genus *Streptomyces*, e.g. *Streptomyces roseofulvus*.

It is also an object of the present invention to provide a protein encoded by a DNA sequence as defined under a) to w) above or with an amino acid sequence as given in SEQ ID NO:1 to 21 and biochemically equivalent variations thereof and a process for the preparation of frenolicins or an biosynthetic intermediate of such frenolicins characterized therein that a cell as specified above is cultured under suitable culture conditions and isolating the frenolicins from the culture or the cells and a process for the preparation of a frenolicin characterized therein that a biosynthetic intermediate obtained by a process as specified above is transformed by chemical or other methods known in the art to such a frenolicin and a process for the preparation of frenolicin B wherein the frenolicins or mixtures of frenolicins obtained according to a process as stated above is/are oxidized to frenolicin B.

It is furthermore an object of the present invention to provide a process for the preparation of a feed composition characterized therein that a process as specified above is effected and the frenolicins obtained are mixed with other feed composition ingredients.

The following figures are used to illustrate the present invention:
Figure 1. Restriction map of the frenolicin gene cluster.
Figure 2. Hypothetical frenolicin biosynthetic pathway with assignment of functions to the various gene subclusters.
Figure 3. Construction of transformation vectors pSSVtsr and pSSVaph.
Figure 4. Sequence of synthetic ermE and tipA promoters.
Figure 5. Construction of vectors for gene expression in *S. roseofulvus*.
The transcription activator gene construct is shown here.

The following definitions are set forth to illustrate and define the meaning and scope of the various terms used to describe the invention herein.

A "host cell" is a cell derived from a procaryotic microorganism which can be used as a recipient for recombinant vectors comprising the DNA sequences, especially the frenolicin gene clusters of this invention. The term includes the progeny of the original cell which has been transformed. The progeny of a single parental cell may not necessarily be completely identical in morphology or in genomic or total DNA complement to the original parent, due to accidental or deliberate mutation. Progeny of the parental cell which are sufficiently similar to the parent to be characterized by the relevant property, such as the presence of the specified DNA sequences are included in the definition.

The term "heterologous" as it relates to nucleic acid sequences such as coding sequences and control sequences, denotes sequences that are not normally associated with a region of a recombinant construct, and/or are not normally associated with a particular cell. Thus, a "heterologous" region of a nucleic acid construct is an identifiable segment of nucleic acid within or attached to another nucleic acid molecule that is not found in association with the other molecule in nature. For example, a heterologous region of a construct could include a coding sequence flanked by sequences not found in association with the coding sequence in nature. Another example of a heterologous coding sequence is a construct where the coding sequence itself is not found in nature (e.g., synthetic sequences having codons different from the native gene). Similarly, a host cell transformed with a construct which is not normally present in the host cell would be considered heterologous for purposes of this invention. Allelic variation or naturally occurring mutational events do not give rise to heterologous DNA, as used herein.

A "coding sequence" is a nucleic acid sequence which is transcribed (in the case of DNA) or translated (in the case of mRNA) into a polypeptide *in vitro* or *in vivo* when placed under the control of appropriate regulatory sequences. The boundaries of the coding sequence are determined by a translation start codon at the 5' (amino) terminus and a translation stop codon at the 3' (carboxy) terminus. A transcription termination sequence will usually be located 3' to the coding sequence.

A "nucleic acid sequence" includes, but is not limited to, procaryotic and eucaryotic mRNA, cDNA from procaryotic and eucaryotic mRNA, genomic DNA, and synthetic DNA and RNA sequences, comprising the natural nucleoside bases adenine, guanine, cytosine, thymidine, and uracil. The term also encompasses sequences having one or more other bases including, but not limited to 4-acetylcytosine, 8-hydroxy-N6-methyladenine, aziridinylcytosine, pseudoisocytosine, 5-(carboxyhydroxylmethyl)uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, and 2,6-diaminopurine.

Nucleic acid "control sequences" refers to translational start and stop codons, promoter sequences, ribosome binding sites, polyadenylation signals, transcription termination sequences, upstream regulatory domains, enhancers, and the like, as necessary and sufficient for the transcription and translation of a given coding sequence in a defined host cell. All of these control sequences need not be present in a recombinant vector so long as those necessary and sufficient for the transcription and translation of the desired gene are present.

"Operably or operatively linked" refers to the configuration of the coding and control sequences so as to perform the desired function. Thus, control sequences operably linked to a coding sequence are capable of effecting the expression of the coding sequence. A coding sequence is operably linked to or under the control of transcriptional regulatory regions in a cell when DNA polymerase will bind the promoter sequence and transcribe the coding sequence into mRNA that can be translated into the encoded protein. The control sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence.

By "selection marker" is meant any genetic marker which can be used to select a population of cells which carry the marker in their genome. Examples of selection markers include: auxotrophic markers by which cells are selected by their ability to grow on minimal media with or without a nutrient or supplement, e.g., thymidine, diaminopimelic acid or biotin; metabolic markers by which cells are selected for their ability to grow on minimal media containing the appropriate sugar as the sole carbon source or the ability of cells to form colored colonies containing the appropriate dyes or chromogenic substrates; and drug resistance markers by which cells are selected by their ability to grow on media containing one or more of the appropriate drugs, e.g., tetracycline, ampicillin, kanamycin, streptomycin or nalidixic acid.

Nucleic acid constructs generally will be provided as transcriptional cassettes. An intron optionally may be included in the construct, preferably 3 100 bp and placed 5' to the coding sequence. Generally it is preferred that the construct not become integrated into the host cell genome and the construct be introduced into the host as part of a non-integrating expression cassette.

The constructs for use in the invention include vectors, transcriptional cassettes, translational cassettes and plasmids. The transcriptional and translational initiation sequences preferably comprise a transcriptional initiation regulatory region (also sometimes referred to as a "promoter") and a translational initiation regulatory region of untranslated 5'-sequences containing a "ribosome binding site," allowing ribosomes to bind to the mRNA and initiate translation at the start codon. It is preferred that all of the transcriptional and translational functional elements of the initiation control region be derived from or obtainable from the same gene. In some embodiments the promoter will be modified by the addition or deletion of sequences, or replaced with alternative sequences. By "obtainable" is intended regulatory sequences sufficiently similar to those of the native gene to provide for the desired specificity of transcription and translation of the DNA sequence of interest. It includes natural and synthetic sequences as well as sequences which may be a combination of synthetic and natural sequences. For the transcriptional initiation region, or promoter element, any region may be used with the proviso that it provides the desired level of transcription of DNA sequence of interest. The transcriptional initiation region may be native to or homologous to the host cell, and/or to the DNA sequence to be transcribed, or foreign or heterologous to the host cell and/or the DNA sequence to be transcribed. By foreign to the host cell is intended that the transcriptional initiation region is not found in the host into which the construct comprising the transcriptional initiation region is to be inserted. By foreign to the DNA sequence is intended a transcriptional initiation region that is not normally associated with the DNA sequence of interest.

Downstream from and under control of the transcriptional initiation regulatory regions is a multiple cloning site for insertion of a nucleic acid sequence of interest which will provide for one or more alterations of host genotype and modulation of host phenotype. Conveniently, the multiple cloning site may be employed for a variety of nucleic acid sequences in an efficient manner. The nucleic acid sequence inserted in the cloning site may have any open reading frame encoding a polypeptide of interest, with the proviso that where the coding sequence encodes a polypeptide of interest, it should lack cruptic splice sites which can block production of appropriate mRNA molecules and/or produce aberrantly spliced or abnormal mRNA molecules. The nucleic acid sequence may be cDNA; it also may be a sequence complementary to a genomic sequence, where the genomic sequence may be one or more of an open reading frame, an intron, a non-coding leader sequence, or any other sequence where the complementary sequence will inhibit transcription, messenger RNA processing, for example splicing, or translation.

The termination region which is employed primarily will be one of convenience, since termination regions appear to be relatively interchangeable. The termination region may be native to the intended nucleic acid sequence of interest, or may be derived from another source.

A transcription vector is constructed so that the particular coding sequence is located in the vector with the appropriate regulatory sequences, the positioning and orientation of the coding sequence with respect to the control sequences being such that the coding sequence is transcribed under the "control" of the control sequences. Modification of the sequences encoding the particular protein of interest may be desirable to achieve this end. For example, in some cases it may be necessary to modify the sequence so that it may be attached to the control sequences with the appropriate orientation; or to maintain the reading frame. The control sequences and other regulatory sequences may be ligated to the coding sequence prior to insertion into a vector. Alternatively, the coding sequence can be cloned directly into an expression vector which already contains the control sequences and an appropriate restriction site which is in reading frame with and under regulatory control of the control sequences.

The term "biochemically equivalent variations" means protein or nucleic acid sequences which differ in some respect from the specific sequences disclosed herein, but nonetheless exhibit the same or substantially the same functionality. In the case of cDNA, for example, this means that modified sequences which contain other nucleic acids than those specifically disclosed are encompassed, provided that the alternate cDNA encodes mRNA which in turn encodes a protein of this invention. Such modifications may involve the substitution of only a few nucleic acids, or many. The modifications may involve substitution of degenerate coding sequences or replacement of one coding sequence with another; introduction of non-natural nucleic acids is contemplated. It is not necessary for the alternate DNA to hybridize with that disclosed herein provided that the functional criterion is met. Similarly, in the case of the proteins of this invention, alterations in the amino acid sequence which do not affect functionality may be made. Such "biochemically equivalent muteins" may involve replacement of one amino acid with another, use of side chain modified or non-natural amino acids, and truncation. The skilled artisan will recognize which sites are most amenable to alteration without affecting the basic function. It is expected that most such alternate sequences will display at least 50% homology to the sequences disclosed herin, preferably at least 75%, and most preferably at least 90%.

The strategy for sequencing the 24 kb frenolicin cluster was adapted from the approach used for large-scale genomic sequencing. Sections of the cluster were randomly digested into 400 - 1800 bp fragments from which libraries were constructed for automated sequencing. The sequence data was collected and manipulated using DNA Star software on a Macintosh Quandra.

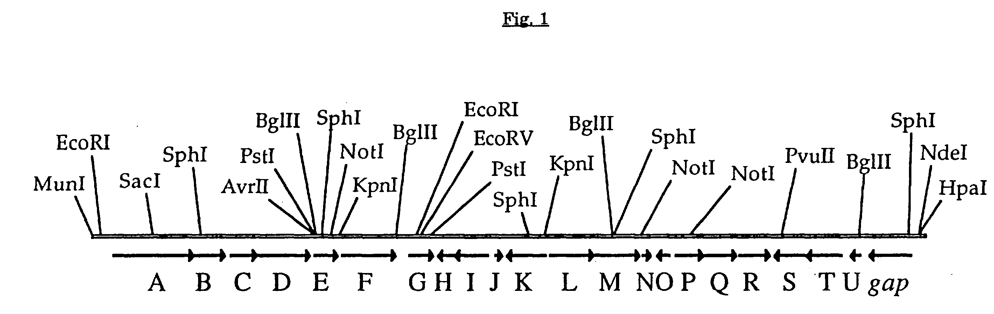

As seen in the restriction map (Figure 1), the 24 kb frenolicin cluster is conveniently segmented by NotI into three large fragments (7.5, 9.0 and 9.5 kb) and one smaller fragment (1.5 kb). The smaller NotI fragment resides in the center of the PKS region of the frenolicin biosynthetic gene cluster and is flanked on both sides by the larger NotI fragments. Each of the NotI fragments from left to right in Figure 1 were subcloned into pBluescript to provide clones denoted Not6, Not7, Not2 and Not3, respectively. The three larger NotI fragments were used to generate separate libraries of MspI fragments for a shotgun sequencing strategy. Sequencing the 3' and 5'-ends of the NotI fragments provided sequence "anchors" to facilitate alignment of the sequence data to the mapped cluster. A random library was constructed from each NotI subclone by gel isolating 0.4 to 1.8 kb fragments after partial digestion with MspI. The fragments were ligated into the AccI site of pBluescript and transformed into a suitable *E. coli* host (DH5α or XL 1-Blue) to form the individual libraries. These libraries were plated and a sufficient number of subclones selected to provide templates for double-stranded sequencing.

Sequencing the templates was accomplished with Applied Biosystems (Foster City, CA) Taq dye-deoxy terminator cycle sequencing reactions which utilize polymerase chain reactions and fluorescently labeled nucleotides. The sequencing gel was read, the data collected, and sequence analyzed with the ABI 373A automated sequencer. As the sequence data was obtained it was aligned with the previously determined sequences to form overlapping, continuous regions of sequence (contigs). As sequence data accumulated, the contigs merged into longer contigs. In most regions both strands were sequenced three or four times, ultimately providing the definitive sequence of the whole cluster.

Genes were located in the sequence as open reading frames (ORFs) having a high frequency of either G or C in the third position of its codons, this being a general property of *Streptomyces* genes (Bibb MJ, et al., *Gene* **30**:157 (1984)). The amino acid sequences of genes located in this way were compared to sequences in the SWISS-PROT database. The nucleotide sequences of the putative genes also were compared to the *Streptomyces* DNA sequences in the GenBank database when the SWISS-PROT database appeared to be lacking certain sequences. In this way genes with sequence similarity to the genes in the frenolicin cluster were found and putative functions assigned.

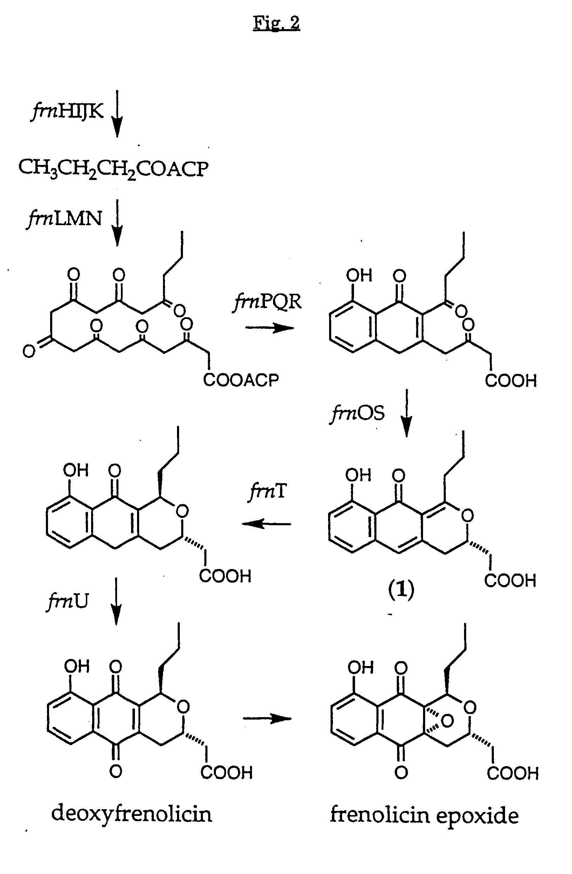

The complete sequence of the frenolicin cluster is provided in SEQ ID NO:22. Starting from the left end of the cluster each gene is discussed below. The hypothetical pathway for frenolicin biosynthesis is shown in Figure 2 with the putative role of each gene product in the pathway indicated. The functions of the indicated genes are summarized in Table 1. The order of genes in the cluster appears to parallel the order of the biosynthetic steps. This feature has been reported previously for macrolide pathways (Donadio S, et al., 1991, *Science* 252:675), but not for aromatic polyketide pathways.

Gene A (bases 636 to 2948 of SEQ ID NO:22 encoding the protein of SEQ ID NO:1) is thought to be the first gene in the cluster since there is a 1 kb non-coding region upstream of it and the 5' end of a partial gene upstream of that region is not homologous to any in the sequence databases. Genes A through D and F comprise a subcluster, which appears to be involved in efflux of frenolicin. The products of genes A through D may represent the components of an ABC transporter system. The characteristic features of ABC transporters include two ATP binding domains, each having two conserved sequence motifs, and two membrane domains, each having six membrane spanning sequences (Higgins CF, *Ann Rev Cell Biol* **8**:67 (1992)). The two ATP-binding domains and the two membrane domains may all be on the same polypeptide, as with the eukaryotic multiple drug resistance proteins, in which case the polypeptide consists of two halves that represent an internal duplication (Chen C et al., 1986, *Cell* 47:381). Bacterial systems often have the different domains on separate polypeptides, although it is likely that in all cases the membrane and ATP-binding components are assembled into a multimeric complex such that the three dimensional positioning of domains is similar in all transporters. Bacterial uptake systems generally have an additional component on the outside of the membrane that binds the substrate to be transported; efflux systems appear to lack this component (Reizer J, et al., *Prot Sci* **1**:1326 (1992)).

Genes A and B (bases 2945 to 3916 of SEQ ID NO:22 encoding the protein of SEQ ID NO:2), and genes C (bases 4020 to 4844 of SEQ ID NO:22 encoding the protein of SEQ ID NO:3) and D (bases 4841 to 6415 of SEQ ID NO:22 encoding the protein of SEQ ID NO:4), are translationally coupled (meaning that the stop codon of one gene is adjacent to the start codon of the next), which is often the case for genes that are coexpressed with a precise stoichiometry. Gene D encodes a soluble 524 amino acid protein containing two ATP binding domains, each with two conserved sequence motifs. The gene D protein consists of two homologous halves, each with homology to the soluble component of the putative efflux system of *S. peucetius*, DrrB (Guilfoyle PG & Hutchinson CR, *Proc Natl Acad Sci* **88**:8553 (1991)), which is proposed to pump the anthracyclines, doxorubicin and daunorubicin, out of the cell. The gene D product also has homology with multidrug resistance pumps from many organisms. Gene C encodes a 274 amino acid protein having the six putative membrane spanning domains typical of such proteins and having good similarity to one of the two membrane components of several bacterial peptide permeases. The protein encoded by gene C also contains a motif often seen in the membrane components of binding protein dependent uptake systems. Gene B, translationally coupled to gene A, also encodes a protein with six putative membrane spanning sequences and shares sequence similarity with the other membrane component of the same bacterial peptide permeases mentioned above. Because genes A and B are translationally coupled, it seems probable that gene A has some role in transport; however, gene A does not have significant homology to other genes in the sequence databases.

Gene F (bases 7344 to 8897 of SEQ ID NO:22 encoding the protein of SEQ ID NO:6) is homologous to the actinorhodin actVA-ORF1 gene (Caballero JL, et al., *Mol Gen Genet* **230**:401 (1991)) and somewhat less homologous to the actII-ORF2 gene (Fernandez-Moreno MA, et al., *Cell* **66**:769 (1991)). Both these genes have been proposed to be involved in actinorhodin efflux. Gene F is also similar to the cmcT gene of the cephamycin C cluster of Nocardia lacamdurans (Coque JJR, et al., *EMBO J* **12**:631 (1993)). Thus gene F may work in concert with genes A, B, C and D or may represent an independent efflux system that is not directly driven by ATP hydrolysis. In between genes D and F is gene E (bases 6533 to 7183 of SEQ ID NO:22 encoding the protein of SEQ ID NO:5), which encodes a 216 amino acid soluble protein with no significant similarity to other genes in the databases.

Gene G (bases 9164 to 10012 of SEQ ID NO:22 encoding the protein of SEQ ID NO:7) encodes a protein with significant similarity to transcription activators in other polyketide gene clusters, including actII-ORF4 in the actinorhodin cluster (Fernandez-Moreno MA, et al., *Cell* **66**:769 (1991)) and dnrI in the daunorubicin cluster (Stutzman-Engwall KJ, et al., *J Bact* **174**:144 (1992)). It probably activates many of the other genes in the cluster through interaction with the putative promoter motif discussed below.

The set of genes H, I, J and K all reside in a subcluster probably involved in the synthesis of the diketide starter unit (e.g., butyryl-ACP, crotonyl-ACP, hydroxybutyryl-ACP or acetoacetyl-ACP) and its transfer to the polyketide synthase to initiate frenolicin biosynthesis. The gene I (SEQ ID NO:9) protein has striking homology to the *E. coli* fabH protein which catalyzes the condensation of acetyl-CoA with malonyl-CoA to form acetoacetyl-ACP, and is believed to initiate fatty acid synthesis in type II systems (Tsay J-T, et al., 1992, *J Biol Chem* 267:6807). Although genes with weak similarity to fabH have been seen in the gene clusters for daunomycin (Ye J, et al., 1994, *J Bacteriol* 176:6270) and doxorubicin (Grimm A, et al., 1994, *Gene* 151:1), this is the first fabH homologue found in an aromatic polyketide gene cluster which has the conserved sequence motif surrounding the active site cysteine. A fabH homologue is not present in the actinorhodin cluster. Although gene M seems to confer loose chain length specificity when coexpressed with other polyketide synthase genes in a heterologous system (McDaniels R, et al., 1993, *J Am Chem Soc* 115:11671), the strains of *S. roseofulvus* discussed herein produce only the 18 carbon frenolicin and not 16 carbon structures such as nanaomycin or kalafungin. Thus, gene I (bases 11628 to 10618 of SEQ ID NO:22 encoding the protein of SEQ ID NO:9) may play a key role in initiating frenolicin biosynthesis and ensuring the 18 carbon length. Translationally coupled to gene I is gene H (bases 10621 to 10105 of SEQ ID NO:22 encoding the protein of SEQ ID NO:8), which has weak similarity to various oxidoreductase geness and to a region of actI-ORF2 of the actinorhodin cluster. The product of gene H may be involved in reduction of acetoacetyl-ACP to butyryl-ACP.

Gene J (bases 11809 to 12066 of SEQ ID NO:22 encoding the protein of SEQ ID NO:10) encodes an acyl carrier protein distinct from that of gene N (see below) which may be used by the gene I protein (along with the gene H protein) to form the starter unit. The protein of gene K (bases 13209 to 12154 of SEQ ID NO:22 encoding the protein of SEQ ID NO:11) is homologous to two putative acyl transferases from the gene clusters for doxorubicin (Grimm A, et al., 1994, *Gene* 151:1) and daunomycin (Ye J, et al., 1994, *J Bacteriol* 176:6270). They all contain the active site motif (GlyHisSer) typical of acyl transferases. The actinorhodin gene cluster does not contain homologs of either genes I or K.

Genes L, M and N encode the core polyketide synthase complex and are homologous to the actI genes of the actinorodin cluster (Fernandez-Moreno MA, et al., 1992, *J Biol Chem* 267:19278). The gene L (bases 13409 to 14686 of SEQ ID NO:22 encoding the protein of SEQ ID NO:12) protein contains active site motifs for β-ketoacyl synthesis and acyl transfer. Gene M (bases 14767 to 16047 of SEQ ID NO:22 encoding the protein of SEQ ID NO:13) is homologous to gene L, but lacks the active site motifs. It is required for a functional polyketide synthase complex. Gene N (bases 16120 to 16371 of SEQ ID NO:22) encodes the protein of SEQ ID NO:14 which is an acyl carrier protein. Based on the proximities of the genes, it would seem that the polyketide synthase uses the gene N acyl carrier protein whereas the fabH homolog uses the gene J acyl carrier protein.

Genes O, P, Q, and R comprise a fourth subcluster. Gene O (bases 16935 to 16453 of SEQ ID NO:22 encoding the protein of SEQ ID NO:15) is homologous to the actVI-ORFA gene of the actinorhodin cluster (Fernandez-Moreno MA, et al., 1994, *J Biol Chem* 269:24854), which is proposed to catalyze a step involving formation and dehydration of a hemiketal structure. Gene P (bases 17088 to 17903 of SEQ ID NO:22) encodes the protein of SEQ ID NO:16 which is homologous to the actIII ketoreductase of the actinorhodin cluster (Hallam SE, et al., 1988, *Gene* 74:305). Gene Q (bases 17903 to 18898 of SEQ ID NO:22) encodes the protein of SEQ ID NO:17 which is homologous to the actVII cyclase/dehydrase of the actinorhodin cluster (Fernandez-Moreno MA, et al., 1992, *J Biol Chem* 267:19278), which is proposed to catalyze the first cyclization reaction. Gene R (bases 18895 to 19839 of SEQ ID NO:22) encodes the protein of SEQ ID NO:18 which is highly similar to the actIV protein of the actinorhodin cluster, a cyclase/dehydrase involved in the second cyclization reaction (Fernandez-Moreno MA, et al., 1992, *J Biol Chem* 267:19278). Since gene R is translationally coupled to gene Q, which is translationally coupled to gene P, these three genes are within a single transcription unit.

Gene S (bases 20907 to 19990 of SEQ ID NO:22 encoding the protein of SEQ ID NO:19) shows weak similarity to the actVA-ORF4, actVI-ORF1 and actVI-ORF2 genes within certain regions. The actVI- ORF1 and actVI-ORF2 gene products are proposed to catalyze a stereospecific keto reduction and a stereospecific enoyl reduction, respectively (Fernandez-Moreno MA, et al., 1994, *J Biol Chem* 269:24854). Since gene T (bases 22094 to 20904 of SEQ ID NO:22 encoding the protein of SEQ ID NO:20) is very similar to actVA-ORF5 (Caballero JL, et al., 1991, *Mol Gen Genet* 230:401), which encodes hydroxylation of the actVA intermediate (Cole SP, et al., 1987, *J Antibiot* 40:340), gene T probably encodes the analogous hydroxlase in frenolicin biosynthesis. Thus, gene S probably encodes one or both of the stereospecific reductions mentioned above.

A putative promoter motif (two or more TGCA sequences separated by exactly 7 bases) lies about 20-50 bp upstream of the start codon of the first ORF of several putative operons in the actinorhodin and frenolicin gene clusters. The presence of this motif only in putative promoter regions of isochromanequinone biosynthetic gene clusters indicates that it probably represents a binding site for a factor involved in transcriptional activation. Genes that are transcribed from promoters in the actinorhodin cluster containing this motif are known to be regulated by the actII-ORF4 gene product (Gramajo HC, et al., 1993, *Mol Microbiol* 7:837). Therefore, gene G in the frenolicin cluster (which is homologous to the actII-ORF4 gene in the actinorhodin cluster) is most likely the regulatory factor involved in activating promoters in the frenolicin cluster that contain this motif. Many of the promoters containing this motif appear able to confer bidirectional transcription.

At the right end of the cluster is a gene with high similarity to those encoding glyceraldehyde-3-phosphate dehydrogenases (G3PDH) from a variety of organisms. It seems unlikely that a G3PDH gene would reside in the cluster so this probably defines the right boundary of the cluster. However, it was observed (Frohlich K-U, et al., 1989, *J Bacteriol* 171:6696) that during pentalenolactone production in *S. arenae*, the pentalenolactone-sensitive G3PDH is replaced by a genetically distinct isoform that is resistant to the product. Thus, it is possible that frenolicin, like pentalenolactone, also inhibits GAPDH, and that a resistant isoform is produced during production.

None of the genes in the sequenced region of the frenolicin cluster clearly encodes either the deoxyfrenolicin epoxidase or the frenolicin B reductase. The only genes that might encode one of these activities are A and E, which do not match anything in the database and have not been assigned a function. Frenolicins are toxic to *Streptomyces* and other bacteria, with frenolicin B being the most toxic, deoxyfrenolicin being less toxic, and frenolicin epoxide even less so. Thus, if either frenolicin B reductase or deoxyfrenolicin epoxidase activity is missing from the frenolicin gene cluster, transformants will be poisoned by the product that results.

To manipulate the frenolicin cluster genes in *S. roseofulvus* and other *Streptomyces*, several *E. coli*/*Streptomyces* shuttle vectors were constructed. For replication in *Streptomyces*, these vectors use the temperature sensitive replicon from plasmid pSG5 (Muth G et al., 1988, *Mol Gen Genet* 211:424; Muth G, et al., 1989, *Mol Gen Genet* 219:341), which has a copy number of about 15 per genome in *S. ghanaensis*, and which has a wide host range. A distinct advantage of this replicon is that growth of transformants at elevated temperature (35 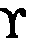 C instead of 28 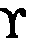 C) prevents autonomous replication, thereby allowing selection for integration of the plasmid into the chromosome, which is required to provide genetically stable strains for industrial production. The backbone for propagation of these vectors in *E. coli* is either Litmus 28 or 38 (New England Biolabs, Beverly, Mass., USA), which are based on the pUC vectors. Since use of single stranded DNA to transform *Streptomyces* hosts has been shown to increase the frequency of homologous integration 10 to 100 times over the same vector in double stranded form (Hillemann D, et al., 1991, *Nucleic Acids Res* 19:777), the Litmus vectors can be used because they include an M13 origin, allowing single stranded DNA to be isolated by infecting the *E. coli* host with M13 helper phage.

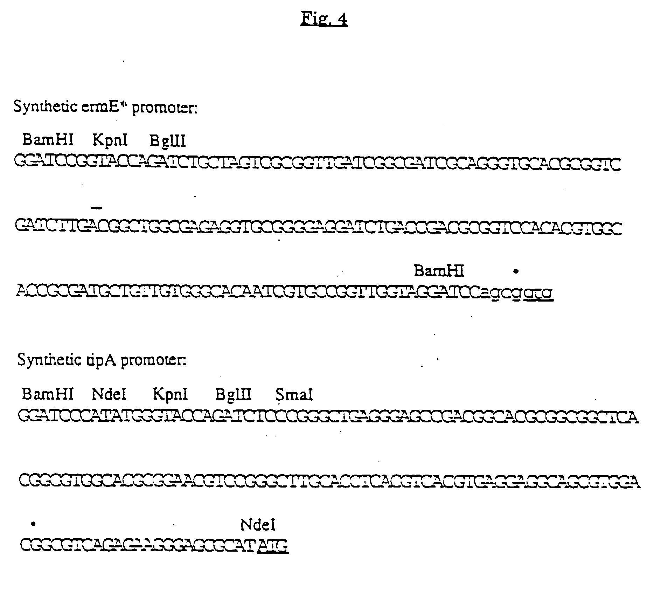

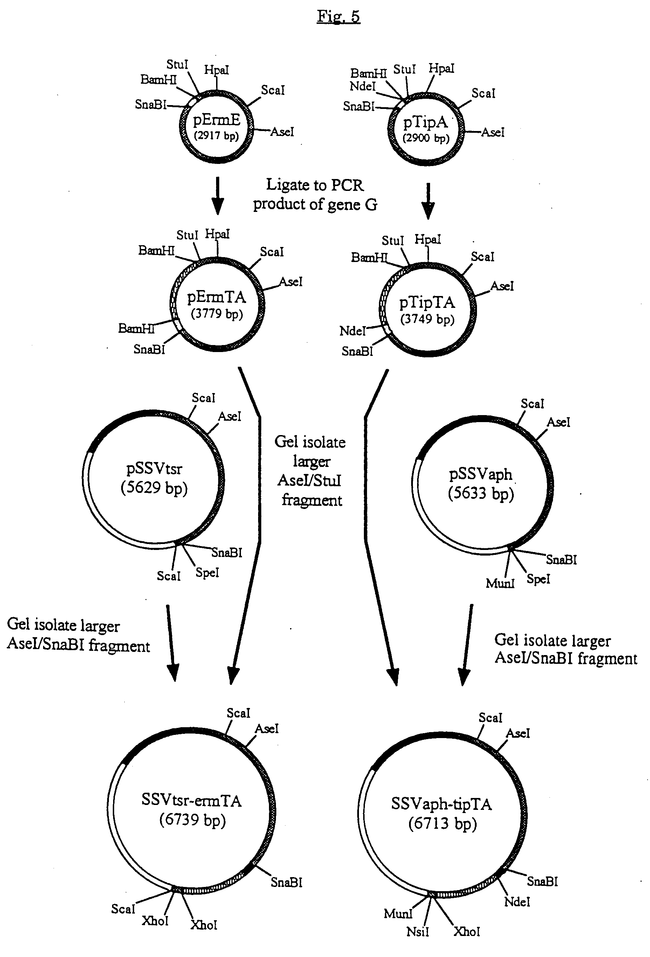

To overexpress DNA sequences of the present invention, especially genes (or transcription units) from the frenolicin cluster, the DNA sequences, gene or operon is/are joined to a strong *Streptomyces* promoter, particularly the tipA and ermE* promoters (Figure 4). To do this, a gene or region of interest is amplified by PCR using primers that supply desired restriction sites and the PCR product is cloned into the appropriate vector, for example pErmE or pTipA (Figure 5). Each promoter-gene chimeric construct is then conveniently moved into the shuttle vectors described above using restriction sites which reconstruct the two halves of the selectable marker (e.g., using the unique AseI site in the ampicillin resistance gene). The gene or region of interest can also be expressed from its native promoter, with higher levels of expression achieved by modifying the promoter or by introducing multiple copies of the construct. If over-expression of any of these genes leads to higher frenolicin titers, these vectors allow one to generate a stable production strain by forcing a homologous integration event by curing the plasmid at elevated temperature while maintaining selection pressure.

Efficient promoter elements for transcription initiation in *Streptomyces* include ermE* and tipA, as well as promoters from many genes known to be expressed at high levels. Regulatory sequences may be joined to a gene of interest using a variety of standard techniques, including site-directed mutagenesis (Kunkel TA, 1985, *Proc. Natl. Acad. Sci.* U.S.A., 82:488-492) to create unique restriction sites at desired positions, synthesis of linker oligonucleotides, or amplification by PCR using primers that create desired restriction sites. In the ideal embodiment, a coding sequence of interest is joined to the regulatory sequences so as to confer initiation of translation at codon #1 of the gene.

Host cells for the recombinant production of frenolicin may be derived from any organism with the capability of harboring a recombinant frenolicin gene cluster or corresponding DNA sequences of the present invention. Thus, the host cells of the present invention can be derived from either prokaryotic or eukaryotic organisms. However, preferred host cells are those constructed from the actinomycetes, a class of mycelial bacteria which are abundant producers of a number of polyketides. A particularly preferred genus for use with the present invention is *Streptomyces*. Thus, for example, *S. ambofaciens*, *S* . *aureofacien*s, *S. avermitilis*, *S. azureus*, *S. cinnamonensis*, *S. coelicolor*, *S. curacoi*, *S. erythraeus*, *S. fradiae*, *S. galilaeus*, *S. glaucescens*, *S. hygroscopicus*, *S.* *lividans*, *S. parvulus*, *S. peucetius*, *S. rimosus*, *S. roseofulvus*, *S. thermotolerans*, *S. violaceoruber*, among others, will provide convenient host cells for the subject invention, with *S. roseofulvus* being preferred. See, e.g., Hopwood, D.A. and Sherman, D.H. *Ann Rev Genet* (1990) 24:37-66; O'Hagan, D. The Polyketide Metabolites (Ellis Horwood Limited, 1991), for a description of various polyketide-producing organisms and their natural products. Such host cells, also used for the purpose to isolate frenolicin gene cluster DNA sequences, can be obtained, e.g. from any known depository authority, e.g. the American Type Culture Collection (ATCC), the Centraalbureau voor Schimmelcultures (CBS) or the Deutsche Sammlung für Mikroorganismen und Zellkulturen GmbH (DSM) or any other depository authority as listed in the Journal "Industrial Property" [(1991) 1, pages 29-40]. For example streptomyces roseofulvus can be obtained from the ATCC as ATCC 19921 or ATCC 19805 or form the CBS as CBS 577.68. In this context it should also be noted that the DNA sequences of the present invention can also be prepared synthetically on the basis of the sequence information as herein disclosed by methods known in the art or described, e.g. in EP 747 483.

The above-described cells can be genetically engineered by deleting any naturally occurring PKS genes therefrom, using standard techniques, such as by homologous recombination. (See, e.g., Khosla, C. et al., 1992, *Molec. Microbiol.* 6:3237). The gene sequences which collectively encode a replacement frenolicin gene cluster or a DNA sequence of the present invention or a functional equivalent, can be inserted into one or more expression vectors, using methods known to those skilled in the art. Expression vectors will include control sequences operably linked to the desired frenolicin coding sequences. Suitable expression systems for use with the present invention include systems which function in eukaryotic and prokaryotic host cells. However, as explained above, prokaryotic systems are preferred, and in particular, systems compatible with *Streptomyces* are of particular interest. Control elements for use in such systems include promoters, optionally containing operator sequences, and ribosome binding sites. Particularly useful promoters include control sequences derived from PKS gene clusters, such as those found 5' of transcription units in the actinorhodin or frenolicin clusters. However, other bacterial promoters, such as those derived from genes that are expressed at high levels will also find use in the present constructs. Examples include promoter sequences derived from genes encoding catabolic enzymes (such as proteases or glycosidases), biosynthetic enzymes (such as those in the tryptophan operon), antibiotic resistance proteins (such as beta-lactamase), or bacteriophage structural proteins. In addition, synthetic promoters, such as the tac promoter (U.S. Patent No. 4,551,433), which do not occur in nature, will also find use in the present constructs. Other regulatory sequences may also be desirable which allow for regulation of expression of the frenolicin replacement sequences relative to the growth of the host cell. Regulatory sequences are known to those skilled in the art, and examples include those which cause the expression of a gene to be turned on or off in response to a chemical or physical stimulus, including the presence of a regulatory compound. Other types of regulatory elements may also be present in the vector, for example, those taken from genes that are expressed during a particular growth phase. Various selectable markers can be included in the recombinant expression vectors. Many markers are known which are useful in selecting for transformed cells and generally comprise a gene whose expression confers a selectable phenotype on transformed cells when the cells are grown in an appropriate selective medium. Such markers include, for example, genes which confer antibiotic resistance or the ability to grow on a minimal medium.

The various frenolicin biosynthetic genes of interest (and functional subunits thereof) or DNA sequences of the present invention can be cloned into one or more recombinant vectors such that each is under the control of its own promoter. Alternatively, genes can be grouped as operons (both those found naturally in the frenolicin cluster or synthetically engineered) under the control of, e.g., a single promoter. Functional subunit sequences can include flanking restriction sites to allow for the easy deletion and/or insertion of other subunits so that hybrid PKSs can be generated. The design of such unique restriction sites is known to those skilled in the art and can be accomplished using the techniques described above, such as site-directed mutagenesis and PCR. Methods for introducing the recombinant vectors of the present invention into suitable hosts are known to those skilled in the art. For *Streptomyces*, protoplasts are typically prepared by digesting the cell wall with lysozyme and the protoplasts are treated with the transforming DNA in combination with various agents that stimulate its uptake, such as polyethylene glycol and divalent cations (Hopwood DA, et al., 1985, Genetic Manipulation of Streptomyces - A Laboratory Manual, John Innes Foundation, Norwich, UK). The protoplasts are subsequently allowed to regenerate into intact cells and transformants are selected. DNA can also be introduced into *Streptomyces* and other bacteria by electroporation. Once the PKS genes are expressed, the polyketide producing colonies can be identified and isolated using known techniques. The polyketides produced by the transformants under appropriate fermentation conditions can then be further processed.

A representative protocol for the fermentation is that described by Iwai et al. in *J. Antibiotics* **31**:959 (1978) and Omura et al. in U.S. Patent No. 4,199,514. A microorganism, e.g. a *Streptomycetes roseofulvus*, transformed with a vector of this invention, may be cultured in the presence of suitable carbon and nitrogen sources, such as cod liver oil, corn oil, glucose, maltose, etc. for carbon, and soybean powders, peptone, yeast, etc. for nitrogen. Fermentation may be carried out by cultivation under aerobic conditions at 20 to 35 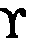 C for about 100 to 150 hours. The culture broth may be separated into cells and filtrate, and the frenolicins extracted from the filtrate with a hydrophobic organic solvent, such as ethyl acetate, butyl acetate, benzene, etc. The frenolicins may then be separated from each other by conventional techniques, such as gel chromatography, and the intermediate compounds (epoxy and deoxyfrenolicins) converted to frenolicin B. Alternatively, the cell-free fermentation broth (preferably obtained by centrifugation) may be hydrogenated under atmospheric pressure at room temperature at pH 8-10 to convert the epoxide to deoxyfrenolicin. After hydrogenation, the pH is adjusted to 5.5 and air blown into the reaction mixture at 60 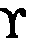 C to oxidize the deoxyfrenolicin to frenolicin B. The frenolicin B is then extracted and precipitated.

### EXAMPLES

### EXAMPLE 1

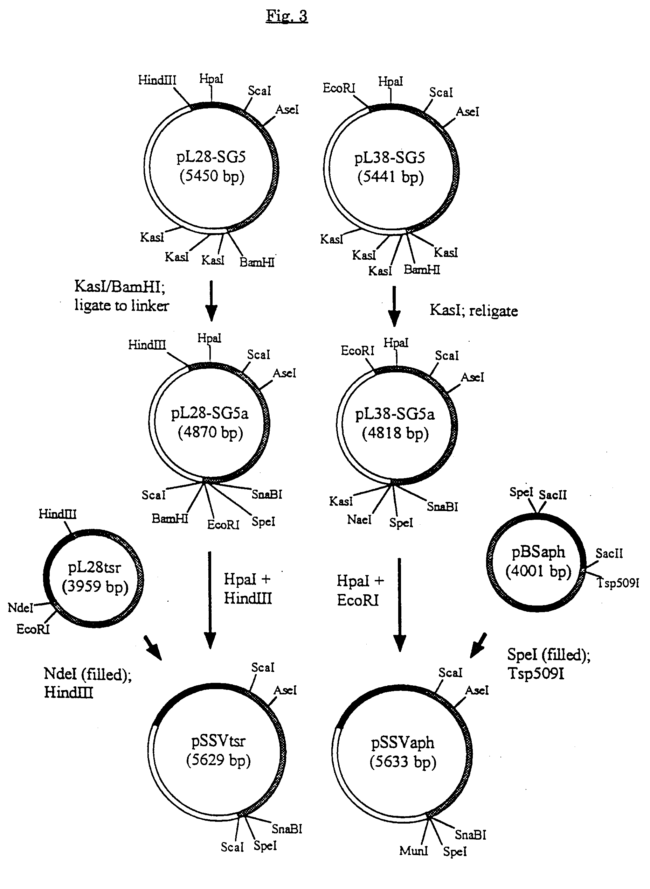

Construction of the *Streptomyces/E. coli* shuttle vectors pSSVtsr and pSSVaph is shown in Figure 3. The pSGS *Streptomyces* replicon and the thiostrepton resistance gene were isolated as cassettes from the vector pGM160 (Muth et al., 1989), which was itself obtained from the vector pCZA168 (Solenberg and Baltz, 1991) by deletion of a transposon as a HindIII insert. Vector pGM160 was digested to completion with BamHI and partially with BclI, and the 2.6kb fragment containing the pSG5 *Streptomyces* replicon was gel isolated and ligated into both Litmus 28 and Litmus 38 (New England Biolabs). The orientation of the SG5 insert that reconstituted a BamHI site proximal to SnaBI in the polylinker of the Litmus vectors was chosen to give plasmids pL28SG 5 and pL38SG5. Based on determination of the minimal replicon of pSG5, as described by Muth et al. (1988), nonessential DNA sequence was deleted to give pL28SG5a and pL38SG5a as follows.

pL38SG5 was digested with KasI and the gel isolated 4.8kb fragment was religated to give pL38SG5a. pL28SG5 was digested with KasI and BamHI and the gel isolated 4.8kb fragment was ligated to the synthetic linker shown below to give pL28SG5a. 
GATCCGCAGTACTGCC
GCGTCATGACGGCGCG .
 The thiostrepton resistance marker was isolated from pCZA168 as a 1.1kb BclI/EcoRI fragment and cloned into the BamHI/EcoRI sites of Litmus 28 to give pL28tsr. The neomycin resistance gene (aph) of *S. fradiae* was isolated as a 1.0 kb SacII fragment from pIJ680 (Hopwood et al., 1985) and cloned into pBluescript SK-(Stratagene, La Jolla, CA, USA) to give pBSaph.

pL28tsr was cut with NdeI and the site was made blunt by filling in with the Klenow fragment of *E. coli* DNA polymerase I. The vector was then cut with HindIII and the resulting 1.1kb fragment was gel isolated. pL28SG5a was cut with HindIII and HpaI and the 5.2kb fragment was gel isolated. These two fragments were then ligated to give pSSVtsr*. pSSVtsr was derived from pSSVtsr* by cutting with BamHI and BglII and religating.

pBSaph was cut with SpeI and the site was made blunt by filling in with the Klenow fragment of *E. coli* DNA polymerase I. The vector was then cut with Tsp509I and the 1.1kb fragment was gel isolated. pL38SG5a was cut with EcoRI and HpaI and the 5.2kb fragment was gel isolated. These two fragments were then ligated to give pSSVaph.

### EXAMPLE 2

The ermE* and tipA promoters were synthesized with convenient restriction sites. A series of overlapping oligonucleotides were synthesized on an ABI 392 DNA synthesizer and subsequently phosphorylated with T4 polynucleotide kinase. These oligonucleotides were then annealed by heating to 90 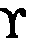 C and cooling slowly. After annealing, the fragments were ligated for four hours at room temperature with T4 ligase and then cut with KpnI and BamHI. The gel isolated fragments were cloned into the KpnI and BamHI sites of Litmus 29 to give vectors pTipA and pErmE (Figure 5).

Various genes in the frenolicin cluster were ampified by PCR, cloned into pTipA and/or pErmE, and then moved into the shuttle vectors by essentially the same procedure to be described in detail for gene G, the transcription activator, below (Figure 5). Vectors for overexpression of chosen genes were thus constructed as described subsequently. Gene G was amplified by PCR using the primers shown below. The resulting PCR products were digested with BamHI (plus NdeI in the case of the product to be ligated into pTipA), gel isolated, and cloned into the BamHI site of pErmE or between the BamHI and NdeI sites of pTipA to give pErmTA and pTipTA, respectively.
Forward (5') PCR primer to amplify gene G for fusion to ermE*
CGGGATCCAGCGGTGGAGATCAAGTACATGGGTCAGTTGACC
Forward (5') PCR primer to amplify gene G for fusion to tipA
CGCATATGGAGATCAAGTACATGGGTCAGTTGACC
Reverse (3') PCR primer to amplify gene G for cloning into pErmE or pTipA
GCGGATCCGTGTCAGTCGTGCGAGCGCGCCGCGGTGGC

The pErmTA and pTipTA vectors were cut with AseI and StuI and the larger (2.8kb) fragment was gel isolated. At the same time, the pSSVtsr and pSSVaph vectors were cut with AseI and SnaBI, and the larger (3.9kb) fragment was gel isolated. The pErmTA and pSSVtsr fragments were ligated together to give SSVtsr-ermTA. The pTipA and pSSVaph fragments were ligated together to give SSVaph-tipTA. For constructs expressing genes via the tipA promoter the neomycin resistance gene is used for selection so that thiostrepton can be used for induction. Because AseI, SnaBI, StuI, BamHI and NdeI sites are absent from the frenolicin cluster, any of the frenolicin genes can be expressed using this strategy.

To prepare vectors designed to allow disruption of specific genes in the cluster, fragments internal to those genes were cloned into Litmus 28 or 38 and the same strategy was used to move the fragments into the shuttle vector. For example, the 1.6kb Sal I fragment spanning most of gene L and the 5' end of gene M was ligated into Litmus 38 and the resulting vector was joined with pSSVtsr to give SSVtsr-deltaLM.

### EXAMPLE 3

Vectors to be used for transformation of *Streptomyces* were propagated in a methylation defective (dam-, dcm-) *E. coli* strain. Vector DNA isolated from this strain was used to transform *S. lividans*, *S. coelicolor*, *S. roseofulvus*. *Streptomyces*, including *S. roseofulvus*, was transformed using a procedure modified from that described previously (Hopwood D, et al., 1985, Genetic Manipulation of Streptomyces - A Laboratory Manual, The John Innes Foundation, Norwich, UK). Twenty five ml of modified YEME media (for each liter: 3g Difco yeast extract, 5g Difco Bacto-peptone, 3g Difco malt extract, 10g glucose, 250g sucrose; after autoclaving, MgCl₂ and glycine are added to 5mM and 0.1% final, respectively) in a 250ml baffled flask was inoculated with a spore suspension and incubated at 30 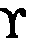 C for 5-8 days with shaking at 220 RPM. The resulting mycelia was washed twice in 10.3% sucrose and resuspended in 4ml lysis buffer (Hopwood et al., 1985, ibid.) containing 0.5 mg/ml lysozyme and 0.5 mg/ml achromopeptidase. The cell walls were digested for 10-15 min at room temperature and monitored microscopically. Remaining mycelial fragments were removed by passing the digest through a cotton plug and the protoplasts were washed twice in fresh protoplast buffer (Hopwood et al., 1985, ibid.) and stored at -80 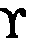 C.

Transformation was accomplished by incubating 50 µl aliquots of the thawed protoplasts with 0.5 - 5 ug plasmid DNA in a solution containing 10 µl of trace elements plus 500 µl of 25% PEG 6000 in transformation buffer (Hopwood et al., 1985, ibid.) for one minute at room temperature. The transformation was mixed with 500 µl of protoplast buffer and 100 - 500 µl aliquots were spread on regeneration plates (CM1-2 containing 10.3 % sucrose). Plates were overlaid after 24 hrs of incubation at 30 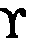 C with soft agar medium containing thiostrepton (to give 30 mg/l final) or neomycin (to give 10 mg/l final) and transformed cells isolated.

**Table 1**

| Gene (SEQ ID NO) | Properties of encoded proteins |
|---|---|
| A (SID:1) | 80kD, non-membrane protein with no strong homologies |
| B (SID:2) | Membrane protein translationally coupled to A; may be involved in transport |
| C (SID:3) | Protein with six membrane spanning domains |
| D (SID:4) | Soluble ATP-binding component of ABC transporter translationally coupled to C |
| E (SID:5) | Function unknown; no homologies in database; not a membrane protein |
| F (SID:6) | Putative efflux pump related to actVA-ORF1; 12-14 membrane spanning domains |
| G (SID:7) | Transcription activator related to actII-ORF4 |
| H (SID:8) | Translationally coupled to gene I |
| I (SID:9) | Homolog of fabH; may synthesize four carbon starter unit |
| J (SID:10) | ACP probably used by the fabH homolog to carry the starter unit |
| K (SID:11) | Acyltransferase; may transfer the starter unit from ACP (J) to the PKS |
| L (SID:12) | PKS ketoacylsynthase subunit |
| M (SID:13) | Additional PKS subunit; so-called "chain length factor" |
| N (SID:14) | The ACP used by the PKS |
| O (SID:15) | Related to actVI-ORFA; possibly a hemiketal dehydrase |
| P (SID:16) | Ketoreductase related to actIII |
| Q (SID:17) | Cyclase/dehydrase related to actVII |
| R (SID:18) | Cyclase/dehydrase related to actIV |
| S (SID:19) | Oxidoreductase weakly related to certain actVI genes |
| T (SID:20) | Quinone-forming hydroxylase related to actVA-ORF5 |

## Claims

1. A DNA sequence comprising at least one of the following DNA sequences:
a) base 636 to 2948 of SEQ ID NO:22.
b) base 2945 to 3916 of SEQ ID NO:22.
c) base 4020 to 4844 of SEQ ID NO:22.
d) base 4841 to 6415 of SEQ ID NO:22.
e) base 6533 to 7183 of SEQ ID NO:22.
f) base 7344 to 8897 of SEQ ID NO:22.
g) base 9164 to 10012 of SEQ ID NO:22.
h) base 10621 to 10105 of SEQ ID NO:22.
i) base 11628 to 10618 of SEQ ID NO:22.
k) base 11809 to 12066 of SEQ ID NO:22.
l) base 13209 to 12154 of SEQ ID NO:22.
m) base 13409 to 14686 of SEQ ID NO:22.
n) base 14767 to 16047 of SEQ ID NO:22.
o) base 16120 to 16371 of SEQ ID NO:22.
p) base 16935 to 16453 of SEQ ID NO:22.
q) base 17088 to 17903 of SEQ ID NO:22.
r) base 17903 to 18898 of SEQ ID NO:22.
s) base 18895 to 19839 of SEQ ID NO:22.
t) base 20907 to 19990 of SEQ ID NO:22.
w) base 22094 to 20904 of SEQ ID NO:22.
or a DNA sequence which codes for a biochemically equivalent variation of a protein encoded by a DNA sequence as specified under a) to w).

2. A DNA sequence according to claim 1 which comprises all DNA sequences as specified under a) to w), preferably organized in the same way as in SEQ ID NO:22, or such DNA sequences wherein at least one of the DNA sequences specified under a) to w) is replaced by a DNA sequence which encodes a protein which is a biochemically equivalent variation thereof.

3. A DNA sequence according to claim 2 which comprises the DNA sequence of SEQ ID NO:22 or a DNA sequence which encodes proteins which are biochemically equivalent variations.

4. A vector comprising DNA sequences as claimed in any one of claims 1 to 3 operatively linked to expression control sequences.

5. A host cell transformed with a vector as claimed in claim 4.

6. The transformed cell of claim 5 wherein the host cell is a member of the genus *Streptomyces*.

7. The transformed cell of claim 6 wherein the host cell is *Streptomyces roseofulvus*.

8. A protein encoded by a DNA sequence as defined under a) to w) in claim 1 or with an amino acid sequence as given in SEQ ID NO:1 to 21 and biochemically equivalent variations thereof.

9. A process for the preparation of frenolicins or an biosynthetic intermediate of such frenolicins characterized therein that a cell as claimed in any one of claim 5 to 7 is cultured under suitable culture conditions and isolating the frenolicins from the culture or the cells.

10. A process for the preparation of a frenolicin characterized therein that a biosynthetic intermediate obtained by a process as claimed in claim 9 is transformed by chemical of other methods known in the art to such a frenolicin.

11. A process for the preparation of frenolicin B wherein the frenolicins or mixtures of frenolicins obtained according to a process as claimed in claim 9 or 10 is/are oxidized to frenolicin B.

12. A process for the preparation of a feed composition characterized therein that a process as claimed in claims 9 to 11 is effected and the frenolicins obtained are mixed with other feed composition ingredients.
